(19) Europäisches Patentamt
European Patent Office
Office européen des brevets

(11) Veröffentlichungsnummer: **0 383 117 B1**

(12) # EUROPÄISCHE PATENTSCHRIFT

(45) Veröffentlichungstag der Patentschrift: **04.05.94**

(51) Int. Cl.$^5$: **C07D 213/74**, A01N 43/40, C07D 213/70, C07D 401/04, C07D 213/63

(21) Anmeldenummer: **90102139.4**

(22) Anmeldetag: **03.02.90**

Die Akte enthält technische Angaben, die nach dem Eingang der Anmeldung eingereicht wurden und die nicht in dieser Patentschrift enthalten sind.

(54) **Pyridyl-substituierte Acrylsäureester.**

(30) Priorität: **17.02.89 DE 3904931**

(43) Veröffentlichungstag der Anmeldung:
**22.08.90 Patentblatt 90/34**

(45) Bekanntmachung des Hinweises auf die Patenterteilung:
**04.05.94 Patentblatt 94/18**

(84) Benannte Vertragsstaaten:
**BE CH DE DK FR GB IT LI NL**

(56) Entgegenhaltungen:
**EP-A- 0 093 908**
**EP-A- 0 243 012**
**EP-A- 0 299 694**

(73) Patentinhaber: **BAYER AG**

**D-51368 Leverkusen(DE)**

(72) Erfinder: **Klausener, Alexander, Dr.**
**Dahlerdyk 173**
**D-4150 Krefeld 1(DE)**
Erfinder: **Kleefeld, Gerd, Dr.**
**Otto-Hahn-Strasse 87**
**D-4000 Duesseldorf 13(DE)**
Erfinder: **Berg, Dieter, Dr.**
**Gellertweg 27**
**D-5600 Wuppertal 1(DE)**
Erfinder: **Dutzmann, Stefan, Dr.**
**Leinenweberweg 33**
**D-4000 Duesseldorf 13(DE)**
Erfinder: **Hänssler, Gerd, Dr.**
**Am Arenzberg 58a**
**D-5090 Leverkusen 3(DE)**

EP 0 383 117 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue pyridyl-substituierte Acrylsäureester, mehrere Verfahren zu ihrer Herstellung, ihre Verwendung in Schädlingsbekämpfungsmittel und teilweise neue Zwischenprodukte.

Es ist bekannt, daß bestimmte substituierte Acrylsäureester, wie beispielsweise die Verbindung 3-Methoxy-2-(2-methylphenyl)-acrylsäuremethylester fungizide Eigenschaften besitzen (vgl. z.B. EP 178 826, EP 243 012).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue pyridyl-substituierte Acrylsäureester der allgemeinen Formel (I)

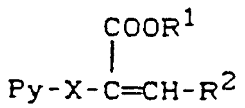

(I)

in welcher

$R^1$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen, -insbesondere Stickstoff, Sauerstoff und/oder Schwefel - im Heteroarylteil und gegebenenfalls 1 bis 4 Kohlenstoffatomen im geradkettigen oder verzweigten Alkylteil;

$R^2$ für Dialkylamino mit jeweils 1 bis 6 Kohlenstoffatomen in den einzelnen Alkylteilen steht oder für einen Rest $-Z-R^3$ steht,

X für Sauerstoff, Schwefel oder für einen Rest

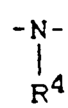

steht und

Py für jeweils einfach bis mehrfach, gleich oder verschieden substituiertes 2-Pyridyl, 3-Pyridyl oder 4-Pyridyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den

einzelnen Alkylteilen, jeweils geradkettiges oder verzweigtes Alkenyl oder Alkinyl mit jeweils 2 bis 8 Kohlenstoffatomen oder jeweils im Arylteil oder im Heteroarylteil gegebenenfalls einfach bis mehrfach, gleich oder verschieden durch Halogen, Alkyl, Alkoxy, Alkylthio, Halogenalkyl, Halogenalkoxy, Halogenalkylthio, Dioxyalkylen, Halogen-substituiertes Dioxyalkylen oder gegebenenfalls substituiertes Phenyl substituiertes Aryl, Aryloxy, Arylthio, Arylcarbonyl, Aralkyl, Aralkenyl, Aralkinyl, Aralkyloxy oder Heteroaryl mit jeweils 6 bis 10 Kohlenstoffatomen im Arylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil bzw. Alkinylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil;
wobei

$R^3$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^4$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach bis mehrfach, gleich oder verschieden substituiertes Aralkyl oder Aryl mit 1 bis 6 Kohlenstoffatomen im geradkettigen oder verzweigten Alkylteil und 6 bis 10 Kohlenstoffatomen im jeweiligen Arylteil steht, wobei als Substituenten im Arylteil jeweils die bei $R^1$ genannten infrage kommen und

Z für Sauerstoff oder Schwefel steht.

Besonders bevorzugt sind Verbindungen der Formel (I), bei welchen gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen pyridylsubstituierten Acrylsäureester der allgemeinen Formel (I),

```
        COOR1
        |
Py-X-C=CH-R2
```

(I)

in welcher

$R^1$, $R^2$, X und Py die oben angegebenen Bedeutungen haben, und

deren Isomere oder Isomerengemische nach einem der im Folgenden beschriebenen Verfahren erhält:

(a) Man erhält pyridyl-substituierte Acrylsäureester der Formel (Ia),

```
        COOR1
        |
Py-X-C=CH-O-R3
```

(Ia)

in welcher

$R^1$, $R^3$, X und Py die oben angegebene Bedeutung haben,

wenn man Hydroxyacrylsäureester oder deren Alkalimetallsalze der Formel (II),

```
        COOR1
        |
Py-X-C=CH-OM
```

(II)

in welcher

M für Wasserstoff oder für ein Alkalimetallkation steht und

$R^1$, X und Py die oben angegebene Bedeutung haben,
mit Alkylierungsmitteln der Formel (III),

$R^3$-$E^1$ (III)

in welcher
$E^1$ für eine elektronenanziehende Abgangsgruppe steht und
$R^3$ die oben angegebene Bedeutung hat,
gegebenenfalls in Gegenwart eines Verdünnungsmittels und gegebenenfalls in Gegenwart eines Reaktionshilfsmittels umsetzt;
(b) man erhält pyridyl-substituierte Acrylsäureester der Formel (Ib),

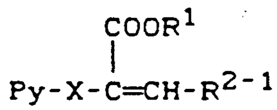
(Ib)

in welcher
$R^{2-1}$ für Dialkylamino steht und
$R^1$, X und Py die oben angegebene Bedeutung haben,
wenn man substituierte Essigsäureester der Formel (IV),

Py-X-$CH_2$-$COOR^1$ (IV)

in welcher
$R^1$, X und Py die oben angegebene Bedeutung haben,
mit Formamiden der Formel (Va),

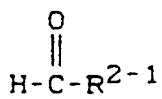
(Va)

in welcher
$R^{2-1}$ die oben angegebene Bedeutung hat,
oder mit Derivaten dieser Formamide der Formel (Vb),

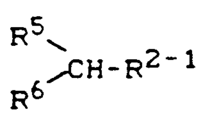
(Vb)

in welcher
$R^5$ und $R^6$ unabhängig von einander jeweils für Alkoxy oder Dialkylamino stehen und
$R^{2-1}$ die oben angegebene Bedeutung hat,
gegebenenfalls in Gegenwart eines Verdünnungsmittels umsetzt;
(c) man erhält pyridyl-substituierte Acrylsäureester der Formel (Ic),

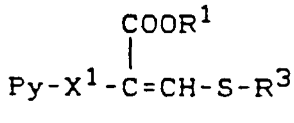
(Ic)

in welcher

$X^1$ für Schwefel oder für einen Rest

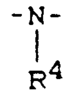

steht

und

$R^1$, $R^3$ und Py die oben angegebene Bedeutung haben,

wenn man Oxalsäurederivate der Formel (VI),

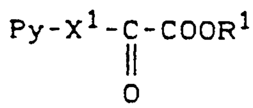

(VI)

in welcher

$R^1$, $X^1$ und Py die oben angegebene Bedeutung haben,

mit metallorganischen Verbindungen der Formel (VII),

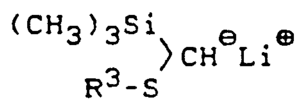

(VII)

in welcher

$R^3$ die oben angegebene Bedeutung hat,

gegebenenfalls in Gegenwart eines Verdünnungsmittels umsetzt;

(d) man erhält pyridyl-substituierte Acrylsäureester der Formel (Id),

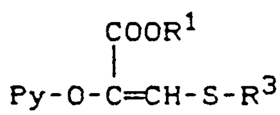

(Id)

in welcher

$R^1$, $R^3$ und Py die oben angegebene Bedeutung haben,

wenn man substituierte Acrylsäureester der Formel (VIII),

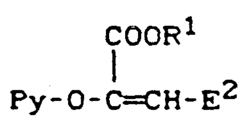

(VIII)

in welcher

$E^2$ für eine elektronenanziehende Abgangsgruppe steht und

$R^1$ und Py die oben angegebene Bedeutung haben,

mit Thiolen der Formel (IX),

$R^3$ - SH (IX)

in welcher

$R^3$ die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen pyridylsubstituierten Acrylsäureester der allgemeinen Formel (I) eine gute Wirkung gegen Schädlinge besitzen.

Überraschenderweise zeigen die erfindungsgemäßen substituierten Acrylsäureester der allgemeinen Formel (I) z.B. eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche chemisch und wirkungsmäßig naheliegende Verbindungen sind.

Besonders bevorzugt sind Verbindungen der Formel (I), bei welchen

$R^1$ für Methyl, Ethyl, n- oder i-Propyl, n-, i-, s-oder t-Butyl oder für gegebenenfalls ein- bis dreifach, gleich oder verschieden substituiertes Benzyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^2$ für Dialkylamino mit jeweils 1 bis 4 Kohlenstoffatomen in den einzelnen Alkylteilen steht oder für einen Rest $-Z-R^3$ steht,

X für Sauerstoff, Schwefel oder für einen Rest

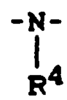

steht und

Py für jeweils gegebenenfalls ein- bis dreifach, gleich oder verschieden substituiertes 2-Pyridyl, 3-Pyridyl oder 4-Pyridyl steht, wobei mindestens ein Substituent jeweils für gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Dioxymethylen oder durch gegebenenfalls ein- bis dreifach, gleich oder verschieden durch Fluor, Chlor, Methyl, Methoxy oder Trifluormethyl substituiertes Phenyl substituiertes Phenyl, Naphthyl, Phenoxy, Phenylthio, Phenylcarbonyl, Benzyl, Phenylethyl, Phenylpropyl, Phenylethenyl, Benzyloxy oder Heteroaryl steht, wobei als Heteroarylreste im einzelnen die folgenden infrage kommen:

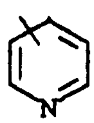 ; 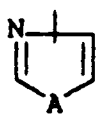 ; 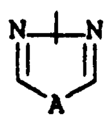 ; 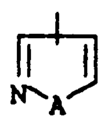 ; 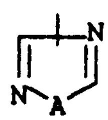 ; 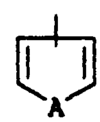 ,

welche gegebenenfalls auch benzannelliert sein können und bei welchen

A jeweils für Sauerstoff, Schwefel oder eine NH-Gruppe steht;

darüberhinaus kommen als weitere Pyridylsubstituenten vorzugsweise die folgenden infrage: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Dimethylcarbamoyl, Diethylcarbamoyl, Allyl, Butenyl oder Propargyl;

und wobei

$R^3$ für Methyl, Ethyl, n- oder i-Propyl, n-, i-, s- oder t-Butyl oder für gegebenenfalls einbis dreifach, gleich oder verschieden substituiertes Benzyl steht, wobei als Substituenten die bei $R^1$ und $R^2$ genannten infrage kommen;

$R^4$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ und $R^2$ genannten infrage kommen und

Z für Sauerstoff oder Schwefel steht.

Ganz besonders bevorzugt sind Verbindungen der Formel (I), bei welchen

$R^1$ für Methyl oder Ethyl steht,

$R^2$ für Methoxy oder Ethoxy steht,

X für Sauerstoff, Schwefel oder für einen Rest

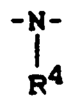

steht,

Py für jeweils gegebenenfalls ein- bis dreifach, gleich oder verschieden substituiertes 2-Pyridyl, 3-Pyridiyl oder 4-Pyridyl steht, wobei mindestens ein Substituent jeweils für gegebenenfalls ein- bis dreifach, gleich oder verschieden durch Fluor, Chlor, Methyl, Ethyl, Methoxy, Trifluormethyl, Dioxymethylen oder Phenyl substituiertes und/oder benzannelliertes Phenyl, Phenoxy, Phenylcarbonyl, Benzyl, Pyridyl, Pyrazolyl, Oxazolyl, Thiazolyl, Thienyl, Furyl, Thiadiazolyl, Oxadiazolyl, Imidazolyl oder Triazolyl steht und wobei als weitere Pyridylsubstituenten jeweils infrage kommen: Fluor, Chlor, Brom, Cyano, Nitro, Methyl, Ethyl, Methoxy, Methylthio, Trifluormethyl, Trifluormethoxy, Trifluormethylthio, Methoxycarbonyl, Ethoxycarbonyl oder Dimethylamino und wobei

$R^4$ für Methyl, Ethyl oder Benzyl steht.

Aryl als solches oder in Zusammensetzungen bedeutet Phenyl oder Naphthyl, insbesondere Phenyl.

Alle aliphatischen Reste als solche oder in Zusammensetzungen sind geradkettig oder verzweigt.

Halogen steht, wenn nicht anders definiert, für Fluor, Chlor, Brom oder Iod, insbesondere für Fluor, Chlor oder Brom.

Im einzelnen seien außer den bei den Herstellungsbeispielen genannten Verbindungen die folgenden pyridylsubstituierten Acrylsäureester der allgemeinen Formel (I) genannt:

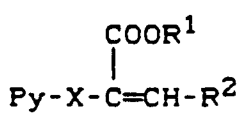

(I)

| Py | $R^1$ | $R^2$ | X |
|---|---|---|---|
| 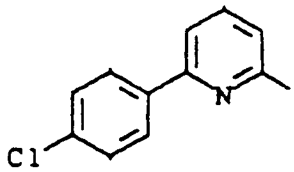 | $CH_3$ | $OCH_3$ | $>N-CH_3$ |
| 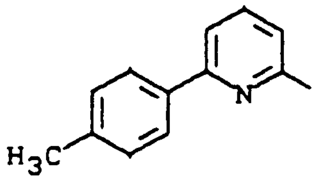 | $CH_3$ | $OCH_3$ | $>N-CH_3$ |
| 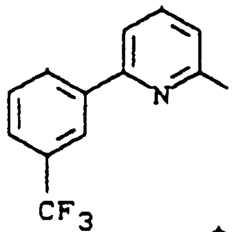 | $CH_3$ | $OCH_3$ | $>N-CH_3$ |
| 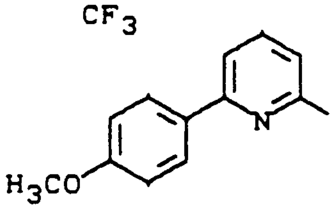 | $CH_3$ | $OCH_3$ | $>N-CH_3$ |
| 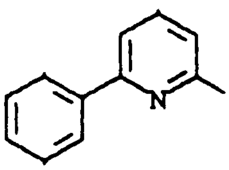 | $C_2H_5$ | $OCH_3$ | $>N-CH_3$ |
| 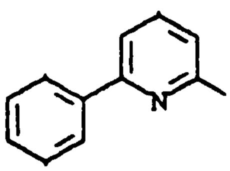 | $C_2H_5$ | $OC_2H_5$ | $>N-CH_3$ |
| 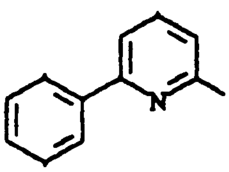 | $CH_3$ | $OCH_3$ | $>N-C_2H_5$ |
| 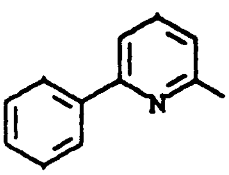 | $CH_3$ | $-N(CH_3)_2$ | $>N-CH_3$ |

| Py | $R^1$ | $R^2$ | X |
|---|---|---|---|
| 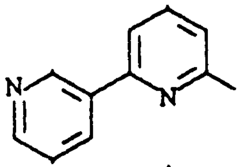 | $CH_3$ | $OC_2H_5$ | $>N-CH_3$ |
| 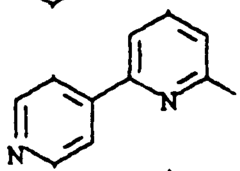 | $CH_3$ | $OCH_3$ | $>N-CH_3$ |
| 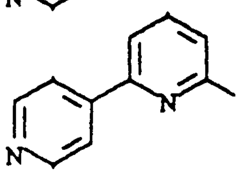 | $CH_3$ | $OC_2H_5$ | $>N-CH_3$ |
| 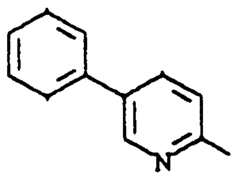 | $CH_3$ | $OCH_3$ | $>N-CH_3$ |
| 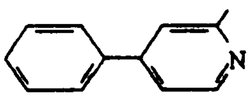 | $CH_3$ | $OCH_3$ | $>N-CH_3$ |
| 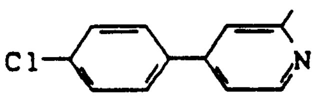 | $CH_3$ | $OCH_3$ | $>N-CH_3$ |
| 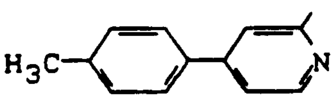 | $CH_3$ | $OCH_3$ | $>N-CH_3$ |
| 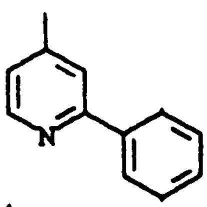 | $CH_3$ | $OCH_3$ | $>N-CH_3$ |
| 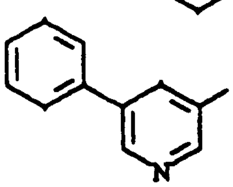 | $CH_3$ | $OCH_3$ | S |

| Py | $R^1$ | $R^2$ | X |
|---|---|---|---|
| 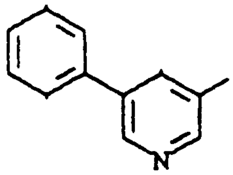 | $CH_3$ | $OCH_3$ | 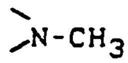 |
| 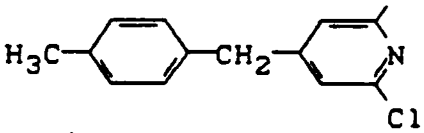 | $CH_3$ | $OCH_3$ | 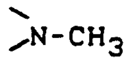 |
| 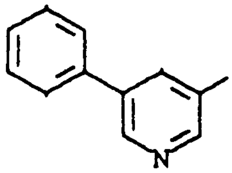 | $CH_3$ | $OCH_3$ | O |
| 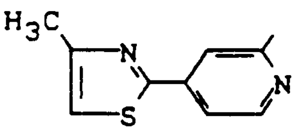 | $CH_3$ | $OCH_3$ | 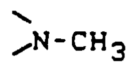 |
| 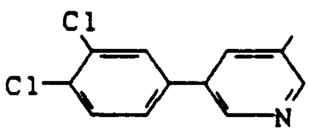 | $CH_3$ | $OCH_3$ | S |
| 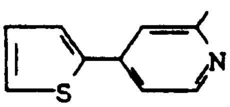 | $CH_3$ | $OCH_3$ | O |
| 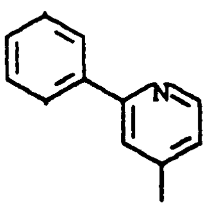 | $CH_3$ | $OCH_3$ | S |
| 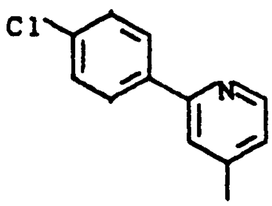 | $CH_3$ | $OCH_3$ | 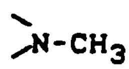 |

| Py | $R^1$ | $R^2$ | X |
|---|---|---|---|
| 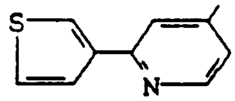 | $CH_3$ | $OCH_3$ | $N-CH_3$ |
| 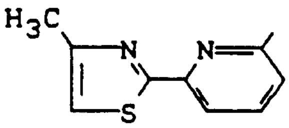 | $CH_3$ | $OCH_3$ | $N-CH_3$ |
| 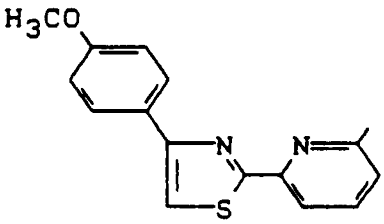 | $CH_3$ | $OCH_3$ | $N-CH_3$ |
| 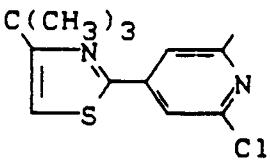 | $CH_3$ | $OCH_3$ | S |
| 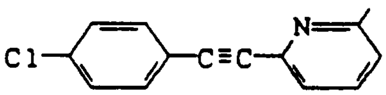 | $CH_3$ | $OCH_3$ | $N-CH_3$ |
| 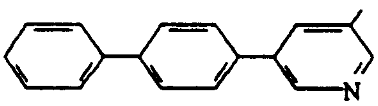 | $CH_3$ | $OCH_3$ | O |
| 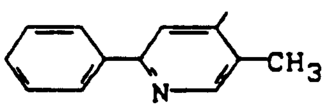 | $CH_3$ | $OCH_3$ | S |
| 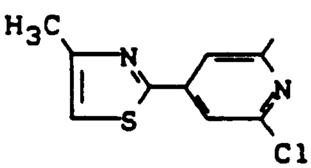 | $CH_3$ | $OCH_3$ | S |
| 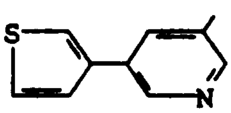 | $CH_3$ | $OCH_3$ | O |

| Py | $R^1$ | $R^2$ | X |
|---|---|---|---|
| 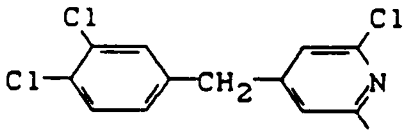 | $CH_3$ | $OCH_3$ | 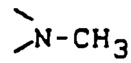 |
| 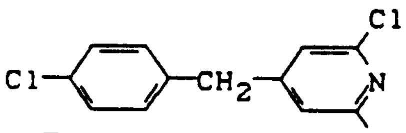 | $CH_3$ | $OCH_3$ | S |
| 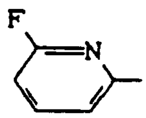 | $CH_3$ | $OCH_3$ | 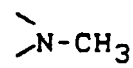 |

Verwendet man beispielsweise 2-{N-[6-(4-Chlorphenyl)-pyridin-2-yl]-N-methylamino}-3-hydroxy-acrylsäuremethylester und Dimethylsulfat als Ausgangsstoffe, so läßt sich der Reaktionsablauf des erfindungsgemäßen Verfahrens (a) durch das folgende Formelschema darstellen:

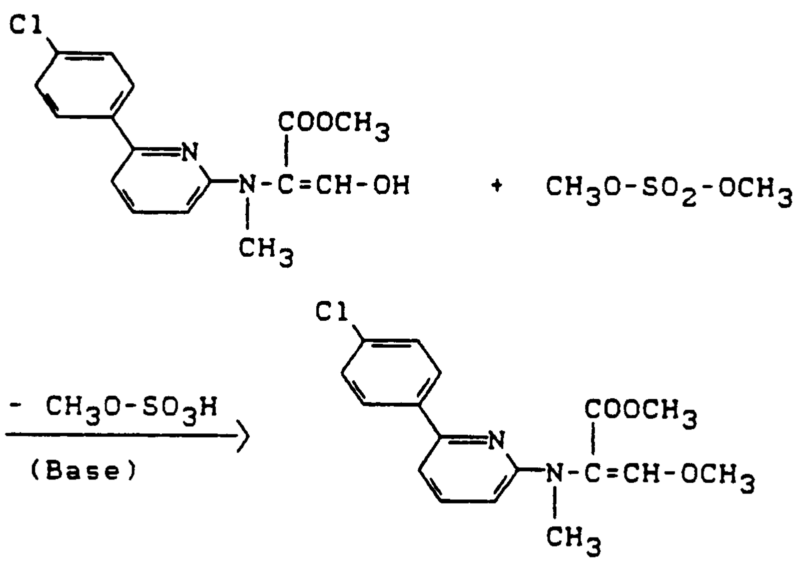


Verwendet man beispielsweise 2-[5-(4-Methylphenyl)-pyridin-2-yloxy]-essigsäuremethylester und Dimethylformamiddimethylacetal als Ausgangsstoffe, so läßt sich der Reaktionsablauf des erfindungsgemäßen Verfahrens (b) durch das folgende Formelschema darstellen:

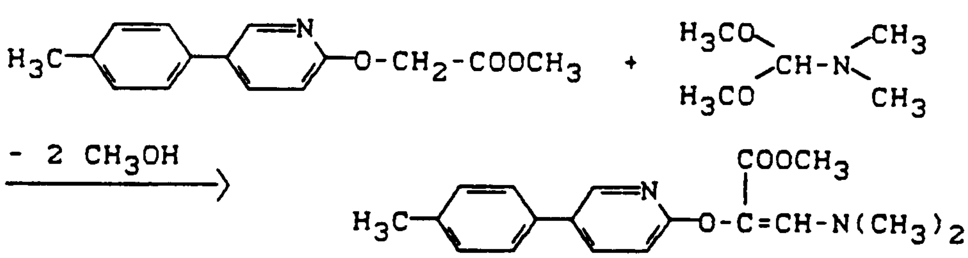

Verwendet man beispielsweise 2-[2-(4-Fluorphenyl)-pyridin-4-ylthio]-2-oxo-essigsäuremethylester und (Methylthio)-(trimethylsilyl)-methylenlithium als Ausgangsstoffe, so läßt sich der Reaktionsablauf des erfindungsgemäßen Verfahrens (c) durch das folgende Formelschema darstellen:

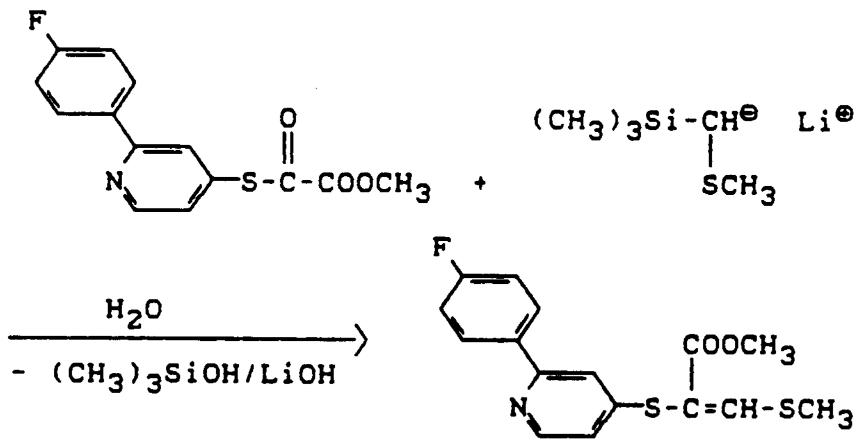


Verwendet man beispielsweise 3-Methylsulfonyloxy-2-(5-phenylpyridin-2-yloxy)-acrylsäuremethylester und Methylmercaptan als Ausgangsstoffe, so läßt sich der Reaktionsablauf des erfindungsgemäßen Verfahrens (d) durch das folgende Formelschema darstellen:

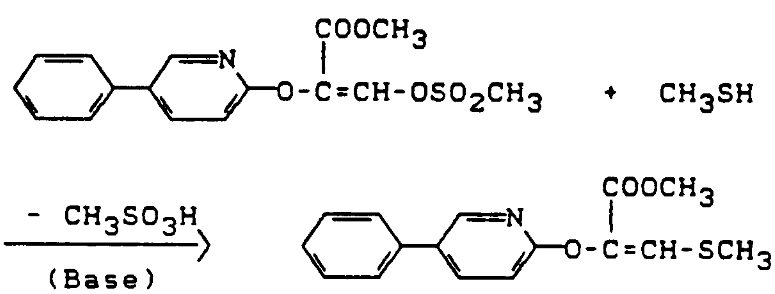


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$ und Py vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Natrium- oder Kaliumkation.

Die Hydroxyacrylsäureester der Formel (II) sind teilweise bekannt (vgl. EP 83 186 oder US 4 468 399).

Noch nicht bekannt und ebenfalls Gegenstand der Erfindung sind Verbindungen der Formel (IIa),

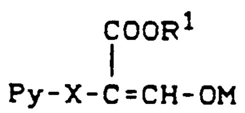

(IIa)

in welcher

$R^1$, Py, X und M die oben angegebene Bedeutung haben,

ausgenommen die Verbindung 2-(6-Methylpyrid-3-yloxy)-3-hydroxyacrylsäureethylester.

Man erhält sie, wenn man substituierte Essigsäureester der Formel (IV),

$Py-X-CH_2-COOR^1$ (IV)

in welcher

$R^1$, Py und X die oben angegebene Bedeutung haben, ausgenommen die Verbindung 2-(6-Methylpyrid-3-yloxy)-essigsäureethylester,

mit Ameisensäureestern der Formel (X),

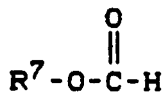

(X)

in welcher

$R^7$ für Alkyl, insbesondere für Methyl oder Ethyl steht,

gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen zwischen - 20 °C und + 50 °C umsetzt.

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester sind durch die Formel (IV) allgemein definiert. In dieser Formel (IV) stehen $R^1$, X und Py vorzugsweise für diejenigen Reste, die bereits im Zusammenhang mit der Beschreibung der erfindungsgemäßen Stoffe der Formel (I) als bevorzugt für diese Substituenten genannt wurden.

Einige der substituierten Essigsäureester der Formel (IV) sind bekannt oder erhältlich in Analogie zu bekannten Verfahren (vgl. z.B. J. Chem. Pharm. Bull. 23, 3008-3010 [1975]; Prakt. Chem. 315, 1175-1182 [1973]; Chimia 28, 235-236 [1974]; Pak J. Sci. Ind. Res. 20, 139-149 [1977]; J. Heterocycl. Chem. 5, 281-283 [1968]; Pol. J. Chem. 53, 2349-2354 [1979]; J. Heterocycl. Chem. 24, 85-89 [1987]; Zh. org. Khim. 20, 1517-1538 [1984]; Zh. Org. Khim. 20, 2002-2011 [1984]; Izv. Akad. Nauk SSSR. Ser. Khim. 1984, 2760-2765; DE 21 03 728; DE 26 37 911; DE 27 09 108; DE 27 25 361; DE 24 25 282; GB 11 61 492 [1969]; EP 182 769; EP 245 230; EP 227 932).

Noch nicht bekannt und ebenfalls Gegenstand der Erfindung sind Verbindungen der Formel (IVa)

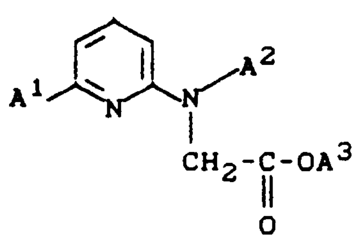

(IVa)

in welcher

$A^1$ für jeweils unsubstituiertes oder einfach bis mehrfach, gleich oder verschieden substituiertes Phenyl, Naphthyl, Pyridyl, Primidinyl, Pyrazinyl, Pyridazinyl, Thienyl, Furanyl, Pyrrolyl, Thiazolyl, Pyrazolyl oder Oxazolyl steht, wobei als Substituenten jeweils genannt seien: Halogen, Methyl, Ethyl, n- oder i-Propyl, n-, i-, s- oder t-Butyl, Methoxy, Ethoxy, n- oder i-Propoxy, n- i-, s- oder t-Butoxy, Halogenalkyl mit 1 oder 2 Kohlenstoffatomen und 1 bis 5 gleichen oder verschiedenen Fluor- oder Chloratomen, Methylthio, Ethylthio, n- oder i-Propylthio, n-, i-, s-oder t-Butylthio, Alkylidendioxy mit 1 bis 3 Kohlenstoffatomen, unsubstituiertes oder einfach bis mehrfach, gleich oder verschieden durch Methyl, Ethyl, Methoxy, Ethoxy, Halogen oder Halogenmethyl mit 1 bis 3

gleichen oder verschiedenen Fluor oder Chloratomen substituiertes Phenyl,

$A^1$ steht weiterhin für unsubstituiertes oder einfach bis mehrfach gleich oder verschieden substituiertes Phenoxy, wobei als Substituenten Halogen, Phenoxy, Methyl, Ethyl, n- oder i-Propyl infrage kommen oder für eine Gruppierung $-C{\equiv}C-A^4$ oder $-CH=CH-A^4$,

$A^2$ für Methyl, Ethyl, n- oder i-Propyl, n-, i-, s-oder t-Butyl steht,

$A^3$ für Methyl, Ethyl, n- oder i-Propyl, n-, i-, s-oder t-Butyl steht und

$A^4$ für unsubstituiertes oder substituiertes Phenyl steht, wobei als Substituenten die weiter oben bei $A^1$ angegebenen Substituenten infrage kommen.

Weiterhin wurde gefunden, daß die neuen Verbindungen der Formel (IVa) eine gute fungizide Wirksamkeit besitzen.

Besonders bevorzugt sind diejenigen Verbindungen der Formel (IVa), in welcher

$A^1$ für jeweils unsubstituiertes oder einfach bis dreifach, gleich oder verschieden substituiertes Phenyl, Naphthyl, Pyridyl, Thienyl oder Thiazolyl steht, wobei als Substituenten jeweils genannt seien:
Fluor, Chlor, Brom, Methyl, Ethyl, Methoxy, Ethoxy, Trifluormethyl, Difluormethyl, Dichlorfluormethyl, Chlordifluormethyl, Methylthio, Methylendioxy, unsubstituiertes oder einfach bis dreifach, gleich oder verschieden durch Methyl, Fluor, Chlor, Methoxy oder Trifluormethyl substituiertes Phenyl,

$A^1$ steht weiterhin für unsubstituiertes oder einfach bis dreifach, gleich oder verschieden substituiertes Phenoxy, wobei als Substituenten Fluor, Chlor, Brom, Phenoxy, Methyl oder Ethyl infrage kommen,
oder für eine Gruppierung $-C{\equiv}C-A^4$ oder $-CH=CH-A^4$,

$A^2$ für Methyl oder Ethyl steht,

$A^3$ für Methyl, Ethyl, n- oder i-Propyl steht und

$A^4$ für unsubstituiertes oder substituiertes Phenyl steht, wobei als Substituenten die weiter oben bei $A^1$ angegebenen Substituenten infrage kommen.

Man erhält die neuen Verbindungen der Formel (IVa)

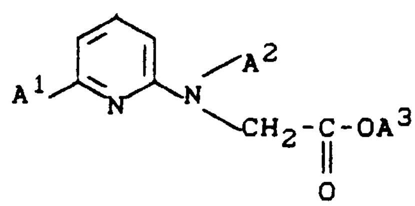

in welcher

$A^1$, $A^2$ und $A^3$ die oben angegebene Bedeutung haben,

wenn man

α) substituierte Halogenpyridine der Formel (XIV)

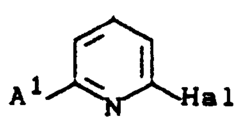

(XIV)

in welcher

$A^1$ die oben angegebene Bedeutung hat und

Hal für Chlor oder Brom steht,

mit Aminosäurederivaten der Formel (XV)

$A^2-NH-CH_2-COOA^3$ (XV)

in welcher

$A^2$ und $A^3$ die oben angegebene Bedeutung haben,

gegebenenfalls in Gegenwart eines Verdünnungsmittels und gegebenenfalls in Gegenwart einer Base

umsetzt,
oder wenn man
β) substituierte Aminopyridine der Formel (XVI)

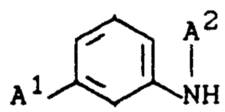

(XVI)

in welcher
$A^1$ und $A^2$ die oben angegebene Bedeutung haben,
mit Halogencarbonsäureestern der Formel (XVII)

$Hal\text{-}CH_2\text{-}COOA^3$ (XVII)

in welcher
$A^3$ die oben angegebene Bedeutung hat und
Hal für Chlor oder Brom steht,
gegebenenfalls in Gegenwart eines Verdünnungsmittels und gegebenenfalls in Gegenwart einer Base umsetzt.

Die Halogenpyridine der Formel (XIV), die Aminosaurederivate der Formel (XV) die Aminopyridine der Formel (XVI) und die Halogencarbonsäureester der Formel (XVII) sind allgemein bekannte Verbindungen der organischen Chemie.

Die Herstellung der erfindungsgemäßen Verbindungen der Formel (IVa) gemäß Verfahrensvariante α) und β) erfolgt vorzugsweise unter Verwendung von Verdünnungsmitteln.

Als solche kommen praktisch alle inerten organischen Lösungsmittel in Frage. Hierzu gehören vorzugsweise Ether, wie Diethyl- und Dibutylether, Glykoldimethylether und Diglykoldimethylether, Tetrahydofuran und Dioxan und Amide, wie z.B. Dimethylformamid, Dimethylacetamid und N-Methyl-pyrrolidon.

Als Basen kommen Alkalicarbonate, wie Natrium- und Kaliumcarbonat oder auch tertiäre Amine, wie beispielsweise Triethylamin, N,N-Dimethylanilin und Pyridin infrage.

Die Reaktionstemperaturen können bei der Herstellung der erfindungsgemäßen Verbindungen der Formel (IVa) gemäß Verfahrensvariante α) und β) in einem größeren Bereich variiert werden. Im allgemeinen arbeitet man zwischen 50° C und +200° C, vorzugsweise zwischen 75° C und +150° C und besonders bevorzugt bei der Rückflußtemperatur des jeweiligen Lösungsmittels.

Zur Durchführung gemäß Verfahrensvariante α) und β) setzt man die Ausgangsstoffe im allgemeinen in äquimolaren Mengen ein. Es ist jedoch auch möglich, eine der beiden jeweils eingesetzten Komponenten in einem größeren Überschuß zu verwenden. Das Reaktionsgemisch wird mehrere Stunden bei der jeweils erforderlichen Temperatur gerührt. Die Aufarbeitung und Isolierung erfolgt nach üblichen Methoden.

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht $R^{2-1}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{2-1}$ steht ganz besonders bevorzugt für Dimethylamino oder Diethylamino.

$R^5$ und $R^6$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest mit jeweils 1 bis 6, insbesondere mit 1 bis 4 Kohlenstoffatomen in den einzelnen geradkettigen oder verzweigten Alkylteilen.

Die Formamide der Formel (Va) und deren Derivate der Formel (Vb) sind allgemein bekannte Verbindungen der organischen Chemie.

Die zur Durchführung des erfindungsgemäßen Verfahrens (c) als Ausgangsstoffe benötigten Oxalsäurederivate sind durch die Formel (VI) allgemein definiert. In dieser Formel (VI) stehen $R^1$ und Py vorzugsweise für diejenigen Reste, die bereits im Zusammenhang mit der Beschreibung der erfindungsgemäßen Stoffe der Formel (I) als bevorzugt für diese Substituenten genannt wurden.

$X^1$ steht vorzugsweise für Schwefel oder für einen Rest

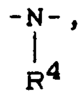

wobei $R^4$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die Oxalsäurederivate der Formel (VI) sind größtenteils bekannt oder erhältlich in Analogie zu bekannten Verfahren (vgl. z. B. J. chem. Soc. C, 1969, 1505-1514; J. med. Chem. 27, 125-128 [1984]; US 4 036 839; US 4 160 100; Synthetic Communications 11, 943 [1981] oder Organic Reactions 26, 1 [1979]), beispielsweise wenn man Oxalester der Formel (XI),

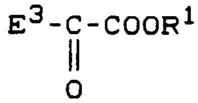

(XI)

in welcher

$E^3$ für Alkoxy oder Halogen insbesondere für Methoxy, Ethoxy oder Chlor steht und

$R^1$ die oben angegebene Bedeutung hat,

mit Heterocyclen der Formel (XII),

Py-$X^1$H (XII)

in welcher

Py und $X^1$ die oben angegebene Bedeutung haben,

gegebenenfalls in Gegenwart eines Verdünnungsmittels, wie beispielsweise Dichlormethan oder Tetrahydrofuran und gegebenenfalls in Gegenwart einer Base, wie beispielsweise n-Butyllithium, Natriumhydrid, Kalium-t-butylat, Triethylamin oder Pyridin bei Temperaturen zwischen - 80 °C und + 80 °C umsetzt.

Oxalester der Formel (XI) sind allgemein bekannte Verbindungen der organischen Chemie.

Heterocyclen der Formel (XII) sind ebenfalls allgemein bekannt oder erhältlich in Analogie zu allgemein bekannten Verfahren.

Die zur Durchführung des erfindungsgemäßen Verfahrens (c) weiterhin als Ausgangsstoffe benötigten metallorganischen Verbindungen sind durch die Formel (VII) allgemein definiert. In dieser Formel (VII) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J. org. Chem. 33, 780 [1968]; J.org. Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert. In dieser Formel (VIII) stehen $R^1$ und Py vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy-oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (IIb),

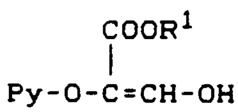

(IIb)

in welcher

$R^1$ und Py die oben angegebene Bedeutung haben,

mit Säurechloriden der Formel (XIII),

$R^8$-Cl (XIII)

in welcher

$R^8$ für einen Acyl- oder Sulfonylrest, insbesondere für einen Acetyl-, einen Methansulfonyl- oder ein p-Toluolsulfonylrest steht,

gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen zwischen - 20 °C und + 120 °C umsetzt.

Säurechloride der Formel (XIII) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$/$C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 30 °C und + 120 °C, vorzugsweise bei Temperaturen zwischen - 20 °C und + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol, vorzugsweise 1.0 bis 2.0 Mol an Reaktionshilfsmittel ein.

Dabei ist es auch möglich, die zur Durchführung des erfindungsgemäßen Verfahrens (a) als Ausgangsverbindungen benötigten Hydroxyacrylsäureester oder deren Alkalimetallsalze der Formel (II) in einer vorgelagerten Reaktion direkt im Reaktionsgefäß herzustellen und anschließend ohne Isolierung gemäß dem erfindungsgemäßen Verfahren (a) weiter umzusetzen (Eintopfvariante). Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 200 °C, vorzugsweise bei Temperaturen zwischen 0 °C und 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein. Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan oder Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen - 100 °C und + 100 °C, vorzugsweise bei Temperaturen zwischen - 80 °C und + 50 °C.

Zur Durchführung des erfindungsgemäßen Verfahrens (c) setzt man pro Mol an Oxalsäurederivat der Formel (VI) im allgemeinen 1.0 bis 1.5 Mol, vorzugsweise 1.0 bis 1.2 Mol an metallorganischer Verbindung der Formel (VII) ein.
Die Reaktionsdurchführung, Aufarbeitung und Isolierung der Reaktionsprodukte erfolgt nach bekannten Verfahren (vgl. z.B. J. org. Chem. 33, 780 [1968]; J. org. Chem. 37; 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und 180 °C, vorzugsweise bei Temperaturen zwischen 0 °C und 150 °C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.
Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Braunspelzigkeit des Weizens (Leptosphaenia nodorum) oder gegen den Erreger der Netzfleckenkrankheit der Gerste (Pyrenophora teres) oder zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen Oomyceten, einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate,

Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

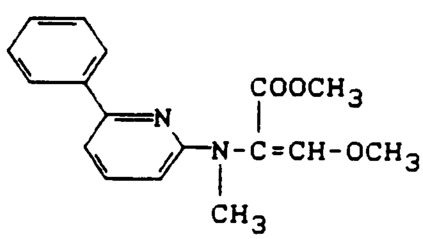

[Verfahren (a) - Eintopfvariante]

Eine Lösung aus 9,0 g (0,032 Mol) N-Methyl-N-(6-phenyl-2-pyridinyl)-aminoessigsäuremethylester in 50 ml Ameisensäuremethylester gibt man tropfenweise bei 5° -10°C unter Rühren und unter einer Argon-Schutzgasatmosphäre im Verlauf von 1 Stunde zu einer Suspension von 2,3 g (0,1 Mol) Natriumhydrid in 40 ml Dimethylformamid, rührt anschließend 20 Stunden bei Raumtemperatur, gibt dann tropfenweise unter Kühlung 11 g (0,09 Mol) Dimethylsulfat und rührt weitere 24 Stunden bei Raumtemperatur.

Zur Aufarbeitung gibt man vorsichtig (zunächst tropfenweise unter Kühlung!) Wasser zu, extrahiert mehrfach mit Essigester, trocknet die vereinigten organischen Phasen über Natriumsulfat, engt im Vakuum ein und reinigt den Rückstand chromatographisch an Kieselgel (Laufmittel: Dichlormethan/Essigester = 15:1).

Man erhält 1,1g (12 % der Theorie) an 3-Methoxy-2-[N-methyl-N-(6-phenyl-2-pyridinyl)-amino]-acrylsäuremethylester vom Schmelzpunkt 89-90°C.

$^1$H-NMR*): ($CDCl_3$/Tetramethylsilan)

$\delta$ = 3,30 (s, 3H); 3,59 (s, 3H); 3,88 (s, 3H); 6,40 (d, 1H); 7,09 (d, 1H); 7,30-7,50 (m, 5H); 8,02 (m, 2H) ppm.

Beispiel 2:

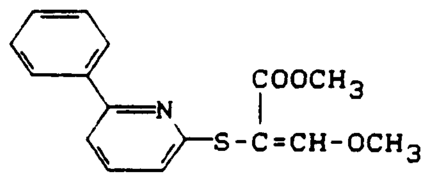

[Verfahren (a)]

Eine Lösung von 5,2 g (0,02 Mol) 2-[(6-Phenyl-2-pyridinyl)-thio]-essigsäuremethylester in 30 ml Ameisensäuremethylester gibt man tropfenweise bei 5 °C bis 10 °C unter Rühren und unter einer Argon-Schutzatmosphäre im Verlauf von 1 Stunde zu einer Suspension von 1,32 g (0,044 Mol) Natriumhydrid in 30 ml Dimethylformamid, rührt anschließend einige Stunden bei Raumtemperatur, stellt dann durch Zugabe von gesättigter wässriger Ammoniumchloridlösung einen pH-Wert von 6 ein, sättigt mit Kochsalz, extrahiert mehrfach mit Essigester, trocknet die vereinigten organischen Phasen tüber Natriumsulfat und engt im Vakuum ein.

Der Rückstand wird in 30 ml Dimethylformamid aufgenommen, mit 10,6 g (0,077 Mol) Kaliumcarbonat und 2,77 g (0,022 Mol) Dimethylsulfat versetzt und 4 Stunden bei Raumtemperatur gerührt. Zur Aufarbeitung gibt man tropfenweise unter Kühlung Wasser zu, extrahiert mehrfach mit Essigester, trocknet die vereinigten organischen Phasen über Natriumsulfat, engt im Vakuum ein und reinigt den Rückstand chromatographisch an Kieselgel (Laufmittel: Dichlormethan/Essigester = 15 : 1).

Man erhält 2,9 g (48 % der Theorie) an 3-Methoxy-2-[(6-phenyl-2-pyridinyl)-thio]-acrylsäure-methylester als zähes Oel.

$^{1}$H-NMR$^{*)}$: ($CDCl_3$/ Tetramethylsilan)

$\delta$ = 3,73 (s, 3H); 3,98 (s, 3H); 7,07 (d, 1H); 7,3-7,5 (m, 4H); 7,56 (pseudo-t, 1H); 7,98 (m, 2H); 8,02 (s, 1H) ppm

In entsprechender Weise und gemäß den allgemeinen Angaben zur Herstellung erhält man die folgenden substituierten Acrylsäureester der allgemeinen Formel (I):

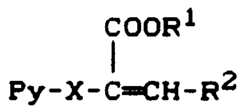

(I)

| Bsp. Nr. | Py | $R^1$ | $R^2$ | X | physikalische Eigenschaften |
|---|---|---|---|---|---|
| 3 | 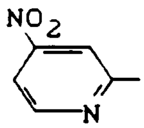 | $CH_3$ | $OCH_3$ | S | Fp:67-72° C |
| 4 | 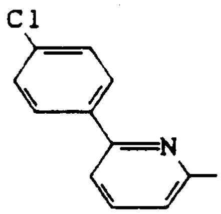 | $CH_3$ | $OCH_3$ | $>N-CH_3$ | Fp: 120° C |
| 5 | 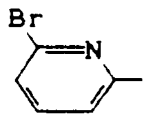 | $CH_3$ | $OCH_3$ | $>N-CH_3$ | Fp:78-79° C |
| 6 | 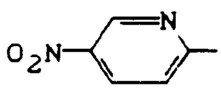 | $CH_3$ | $OCH_3$ | $>N-CH_3$ | MS: 267($M^{\oplus}$) 236;190 |
| 7 | 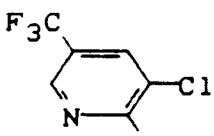 | $CH_3$ | $OCH_3$ | $>N-CH_3$ | $^1$H-NMR*): 3,24; 3,75; 3,80; 7,26; 7,61; 8,234 |
| 8 | 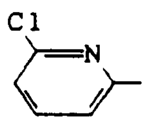 | $CH_3$ | $OCH_3$ | S | Fp: 58-60° C |
| 9 | 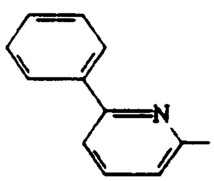 | $C_2H_5$ | $OCH_3$ | $>N-CH_3$ | $^1$H-NMR*): 1,15; 3,30; 3,85; 4,17; 6,40; 7,07; 7,3-7,5;8,02 |
| 10 | 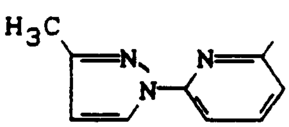 | $CH_3$ | $OCH_3$ | S | Fp:95-96° C |
| 11 | 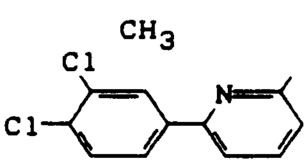 | $CH_3$ | $OCH_3$ | $>N-CH_3$ | $^1$H-NMR*): 3,74; 4,0; 7,10; 7,39; 7,49; 7,55; 7,79; 8,00; 8,09 |

| Bsp. Nr. | Py | $R^1$ | $R^2$ | X | physikalische Eigenschaften |
|---|---|---|---|---|---|
| 12 | 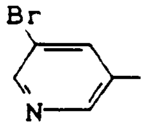 | $CH_3$ | $OCH_3$ | O | Fp: 62° C |
| 13 | 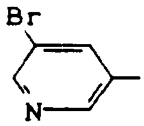 | $CH_3$ | $OCH_3$ | O | Fp: 69° C (Z-Form) |
| 14 | 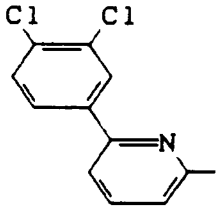 | $CH_3$ | $OCH_3$ | S | $^1$H-NMR*): 3,74; 4,0; 7,10; 7,39; 7,49, 7,55; 7,79; 8,00 |
| 15 | 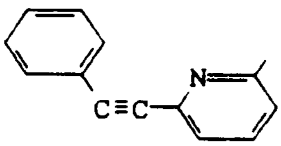 | $CH_3$ | $OCH_3$ | 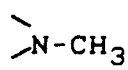 | $^1$H-NMR*): 3,26; 3,7; 3,9; 6,3-7,6 |
| 16 | 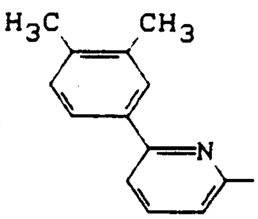 | $CH_3$ | $OCH_3$ | 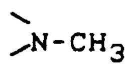 | Fp:100-105° C |
| 17 | 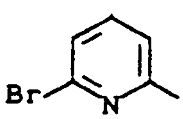 | $CH_3$ | 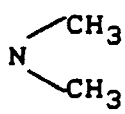 | 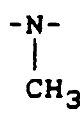 | Fp: 107° C |

| Bsp. Nr. | Py | $R^1$ | $R^2$ | X | physikalische Eigenschaften |
|---|---|---|---|---|---|
| 18 | 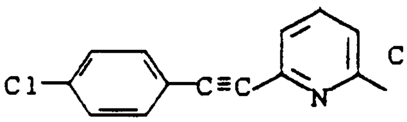 | $CH_3$ | 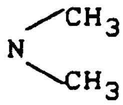 | 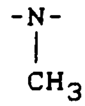 | Fp: 152-153° C |
| 19 | 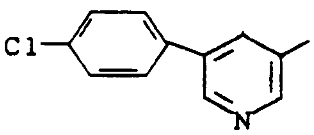 | $CH_3$ | $OCH_3$ | O | Fp: 62-63° C |
| 20 | 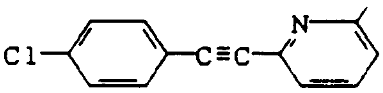 | $CH_3$ | $OCH_3$ | 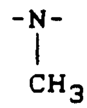 | |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen:

Beispiel IV-1:

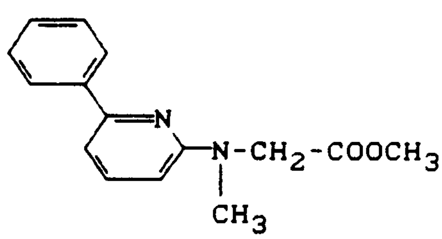

Zu 13 g (0,05 Mol) N-(6-Brom-2-pyridinyl)-N-methylaminoessigsäuremethylester und 1,0 g Tetrakis-(triphenylphosphin)-Palladium(O) in 100 ml Benzol und 50 ml 2-molarer wässriger Natriumcarbonatlösung gibt man unter kräftigem Rühren bei Raumtemperatur im Verlauf von 30 Minuten, tropfenweise 6,75 g (0,0555 Mol) Phenylboronsäure in wenig Methanol gelöst, rührt anschließend bei Rückflußtemperatur bis im Gaschromatogramm kein Ausgangsprodukt mehr nachweisbar ist, kühlt ab, trennt die organische Phase ab, wäscht mit Wasser, trocknet über Natriumsulfat, engt ein und reinigt den Rückstand durch Säulenchromatographie an Kieselgel (Laufmittel: Dichlormethan/n-Hexan = 1:1).

Man erhält 11,7 g (91 % der Theorie) an N-(6-Phenyl-2-pyridinyl)-N-methylamino-essigsäuremethylester als Öl.
$^{1}$H-NMR (CDCl$_3$/Tetramethylsilan):
$\delta$ = 3,18 (s, 3H); 3,72 (s, 3H); 4,41 (s, 2H); 6,54 (d, 1H); 7,11 (d, 1H); 7,30-7,48 (m, 3H); 7,57 (dd, 1H); 7,98 (m, 2H) ppm.

Beispiel IV-2:

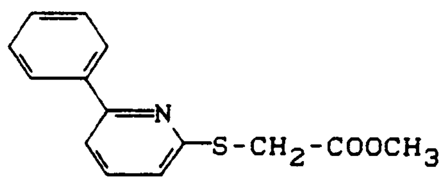

Zu einem Gemisch aus 13,1 g (0,05 Mol) 2-(6-Brom-2-pyridinylthio)-essigsäuremethylester und 2,5 g Tetrakis(triphenylphosphin)-Palladium(O) in 200 ml Benzol und 100 ml 2-molarer wässriger Natriumcarbonatlösung gibt man unter kräftigem Rühren bei Raumtemperatur im Verlauf von 30 Minuten tropfenweise 13,5 g (0,11 Mol) Phenylboronsäure in wenig Methanol gelöst, rührt anschließend 15 Stunden bei Rückflußtemperatur, kühlt ab, trennt die organische Phase ab, wäscht mit Wasser, trocknet über Natriumsulfat und reinigt den Rückstand durch Säulenchromatographie an Kieselgel (Laufmittel: Dichlormethan/n-Hexan = 1:1).

Man erhält 10,3 g (79,4 % der Theorie) an 2-(6-Phenyl-2-pyridinylthio)-essigsäuremethylester als Öl.
$^{1}$H-NMR$^{*)}$: (CDCl$_3$/Tetramethylsilan):
$\delta$ = 3,73 (s, 3H); 4,06 (s, 2H); 7,17 (d, 1H); 7,35 - 7,5 (m, 4H); 7,58 (t, 1H); 8,0 (m, 2H).

Beispiel IV-3:

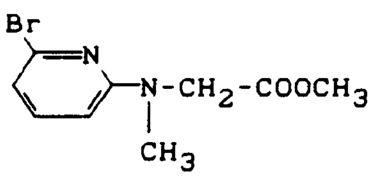

Ein Mischung aus 17 g (0,12 Mol) N-Methylglycinmethylester-Hydrochlorid, 29 g (0,12 Mol) 2,6-Dibrompyridin, 27 g (0,2 Mol) Kaliumcarbonat und 200 ml Dioxan wird 48 Stunden auf Rückflußtemperatur erhitzt, anschließend filtriert, das Filtrat eingeengt und durch Chromatographie an Kieselgel (Laufmittel: Dichlormethan/n-Hexan = 1:1) gereinigt.

Man erhält 13,1 g (41 % der Theorie) an N-(6-Brom-2-pyridinyl)-N-methylaminoessigsäuremethylester vom Schmelzpunkt 47-48 °C.
$^{1}$H-NMR$^{*)}$: (CDCl$_3$/Tetramethylsilan):
$\delta$ = 3,10 (s, 3H); 3,75 (s, 3H); 4,35 (s, 2H); 6,46 (d, 1H); 6,75 (d, 1H); 7,31 (pseudo-t, 1H) ppm.

Beispiel IV-4:

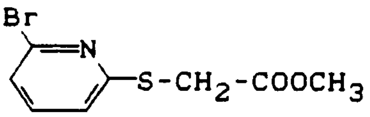

Eine Mischung aus 23,7 g (0,1 Mol) 2,6-Dibrompyridin, 10,6 g (0,1 Mol) Thioessigsäuremethylester, 10 g (0,075 Mol) Kaliumcarbonat und 100 ml Dioxan wird 20 Stunden unter Rühren und unter einer Argon-Schutzgasatmosphäre auf Rückflußtemperatur erhitzt, anschließend abgekühlt, mit Wasser versetzt, mit Essigester extrahiert, über Natriumsulfat getrocknet und im Vakuum eingeengt. Der Rückstand wird aus Diisopropylether/n-Hexan umkristallisiert.

Man erhält 19,4 g (74 % der Theorie) an 2-(6-Brom-2-pyridinylthio)-essigsäuremethylester vom Schmelzpunkt 32-34 °C.

Anwendungsbeispiele

In den folgenden Anwendungsbeispielen wurde die nachstehend aufgeführte Verbindung als Vergleichsubstanz eingesetzt:

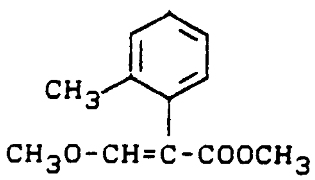

(A)

3-Methoxy-2-(2-methylphenyl)-acrylsäuremethylester
(bekannt aus EP 178 826).

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 2, 4, 5 und 9.

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1 und 5.

**Patentansprüche**

**1.** Pyridyl-substituierte Acrylsäureester der allgemeinen Formel (I)

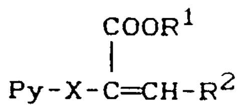

(I)

in welcher

$R^1$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen im Heteroarylteil und gegebenenfalls 1 bis 4 Kohlenstoffatomen im geradkettigen oder verzweigten Alkylteil;

$R^2$ für Dialkylamino mit jeweils 1 bis 6 Kohlenstoffatomen in den einzelnen Alkylteilen steht oder für einen Rest $-Z-R^3$ steht,

X für Sauerstoff, Schwefel oder für einen Rest

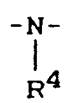

steht und

Py für jeweils einfach bis mehrfach, gleich oder verschieden substituiertes 2-Pyridyl, 3-Pyridyl

oder 4-Pyridyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen oder jeweils im Arylteil oder im Heteroarylteil gegebenenfalls einfach bis mehrfach, gleich oder verschieden durch Halogen, Alkyl, Alkoxy, Alkylthio, Halogenalkyl, Halogenalkoxy, Halogenalkylthio, Dioxyalkylen, Halogen-substituiertes Dioxyalkylen oder gegebenenfalls substituiertes Phenyl substituiertes Aryl, Aryloxy, Arylthio, Arylcarbonyl, Aralkyl, Aralkenyl, Aralkinyl, Aralkyloxy oder Heteroaryl mit jeweils 6 bis 10 Kohlenstoffatomen im Arylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil bzw. Alkinylteil oder mit 2 bis 9 Kohlenstoffatomen und 1 bis 4 gleichen oder verschiedenen Heteroatomen im Heteroarylteil;

wobei

$R^3$ für geradkettiges oder verzweigtes Alkyl mit 1 bis 6 Kohlenstoffatomen oder für gegebenenfalls im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kömmen,

$R^4$ für Wasserstoff, für geradkettiges oder verzweigtes Alkyl mit 1 bis 6 Kohlenstoffatomen, für geradkettiges oder verzweigtes Alkanoyl mit 1 bis 6 Kohlenstoffatomen im Alkylteil oder für jeweils gegebenenfalls im Arylteil einfach bis mehrfach, gleich oder verschieden substituiertes Aralkyl oder Aryl mit 1 bis 6 Kohlenstoffatomen im geradkettigen oder verzweigten Alkylteil und mit 6 bis 10 Kohlenstoffatomen im jeweiligen Arylteil steht, wobei als Substituenten im Arylteil jeweils die bei $R^1$ genannten infrage kommen und

Z für Sauerstoff oder Schwefel steht.

**2.** Pyridyl-substituierte Acrylsäureester der Formel (I) gemaß Anspruch 1, worin

$R^1$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy oder Benzyloxy,

$R^2$ für Dialkylamino mit jeweils 1 bis 4 Kohlenstoffatomen in den einzelnen Alkylteilen steht oder für einen Rest $-Z-R^3$ steht,

X für Sauerstoff, Schwefel oder für einen Rest

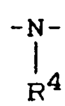

steht und

Py für jeweils gegebenenfalls ein- bis dreifach, gleich oder verschieden substituiertes 2-Pyridyl, 3-Pyridyl oder 4-Pyridyl steht, wobei mindestens ein Substituent jeweils für gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Dioxymethylen oder durch gegebenenfalls ein- bis dreifach, gleich oder verschieden durch Fluor, Chlor, Methyl, Methoxy oder Trifluormethyl substituiertes Phenyl substituiertes Phenyl, Naphthyl, Phenoxy, Phenylthio, Phenylcarbonyl, Benzyl, Phenylethyl, Phenylpropyl, Phenylethenyl, Benzyloxy oder Heteroaryl steht, wobei als Heteroarylreste im einzelnen die folgenden infrage kommen:

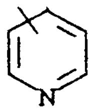 ; 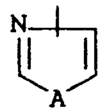 ; 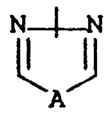 ; 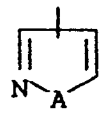 ; 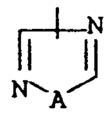 ; 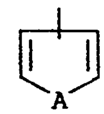 ,

welche gegebenenfalls auch benzannelliert sein können und bei welchen

A jeweils für Sauerstoff, Schwefel oder eine NH-Gruppe steht, weitere Pyridylsubstituenten sind: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Dimethylcarbamoyl, Diethylcarbamoyl, Allyl, Butenyl oder Propargyl; wobei

$R^3$ für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten die bei $R^1$ und $R^2$ genannten infrage kommen;

$R^4$ für Wasserstoff, Methyl, Ethyl, n- oder i-Propyl, n-, i-, s- oder t-Butyl, für Acetyl, Propionyl, n- oder i-Butyryl oder für jeweils gegebenenfalls ein- bis dreifach, gleich oder verschieden substituiertes Benzyl oder Phenyl steht, wobei als Substituenten die bei $R^1$ und $R^2$ genannten infrage kommen und

Z für Sauerstoff oder Schwefel steht.

**3.** Pyridyl-substituierte Acrylsäure der Formel (I) gemäß Anspruch 1, worin

$R^1$ für Methyl oder Ethyl steht,

$R^2$ für Methoxy oder Ethoxy steht,

X für Sauerstoff, Schwefel oder für einen Rest

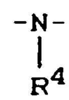

steht,

Py für jeweils gegebenenfalls ein- bis dreifach, gleich oder verschieden substituiertes 2-Pyridyl, 3-Pyridiyl oder 4-Pyridyl steht, wobei mindestens ein Substituent jeweils für gegebenenfalls ein- bis dreifach, gleich oder verschieden durch Fluor, Chlor, Methyl, Ethyl, Methoxy, Trifluormethyl, Dioxymethylen oder Phenyl substituiertes und/oder benzannelliertes Phenyl, Phenoxy, Phenylcarbonvl, Benzyl, Pyridyl, Pyrazolyl, Oxazolyl, Thiazolyl, Thienyl, Furyl, Thiadiazolyl, Oxadiazolyl, Imidazolyl oder Triazolyl steht und wobei als weitere Pyridylsubstituenten jeweils infrage kommen: Fluor, Chlor, Brom, Cyano, Nitro, Methyl, Ethyl, Methoxy, Methylthio, Trifluormethyl, Trifluormethoxy, Trifluormethylthio, Methoxycarbonyl, Ethoxycarbonyl oder Dimethylamino und wobei

$R^4$ für Methyl, Ethyl oder Benzyl steht.

**4.** Verfahren zur Herstellung von pyridyl-substituierten Acrylsäureestern der allgemeinen Formel (I),

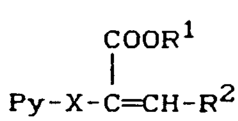 (I)

in welcher

$R^1$, $R^2$, X und Py die in Anspruch 1 angegebene Bedeutung haben,

dadurch gekennzeichnet, daß man

(a) pyridyl-substituierte Acrylsäureester der Formel (Ia),

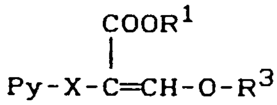

(Ia)

in welcher

$R^1$, $R^3$, X und Py die oben angegebene Bedeutung haben, erhält,

indem man Hydroxyacrylsäureester oder deren Alkalimetallsalze der Formel (II),

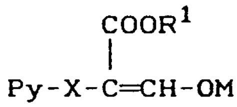

(II)

in welcher

M für Wasserstoff oder für ein Alkalimetallkation steht und

$R^1$, X und Py die oben angegebene Bedeutung haben,

mit Alkylierungsmitteln der Formel (III),

$R^3$-$E^1$ (III)

in welcher

$E^1$ für eine elektronenanziehende Abgangsgruppe steht und

$R^3$ die oben angegebene Bedeutung hat,

gegebenenfalls in Gegenwart eines Verdünnungsmittels und gegebenenfalls in Gegenwart eines Reaktionshilfsmittels umsetzt oder

(b) pyridyl-substituierte Acrylsäureester der Formel (Ib),

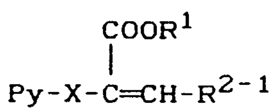

(Ib)

in welcher

$R^{2-1}$ für Dialkylamino steht und

$R^1$, X und Py die oben angegebene Bedeutung haben, erhält,

indem man substituierte Essigsäureester der Formel (IV),

Py-X-$CH_2$-$COOR^1$ (IV)

in welcher

$R^1$, X und Py die oben angegebene Bedeutung haben,

mit Formamiden der Formel (Va),

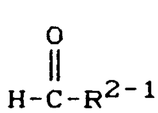

(Va)

in welcher

$R^{2-1}$ die oben angegebene Bedeutung hat,

oder mit Derivaten dieser Formamide der Formel (Vb),

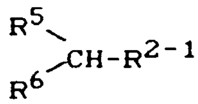 (Vb)

in welcher

$R^5$ und $R^6$ unabhängig von einander jeweils für Alkoxy oder Dialkylamino stehen und

$R^{2-1}$ die oben angegebene Bedeutung hat,

gegebenenfalls in Gegenwart eines Verdünnungsmittels umsetzt oder

(c) pyridyl-substituierte Acrylsäureester der Formel (Ic),

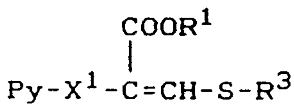 (Ic)

in welcher

$X^1$ für Schwefel oder für einen Rest

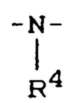

steht

und

$R^1$, $R^3$ und Py die oben angegebene Bedeutung haben, erhält,

indem man Oxalsäurederivate der Formel (VI),

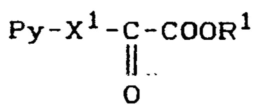 (VI)

in welcher

$R^1$, $X^1$ und Py die oben angegebene Bedeutung haben,

mit metallorganischen Verbindungen der Formel (VII),

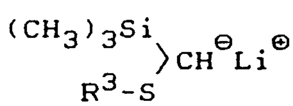 (VII)

in welcher

$R^3$ die oben angegebene Bedeutung hat,

gegebenenfalls in Gegenwart eines Verdünnungsmittels umsetzt oder

(d) pyridyl-substituierte Acrylsäureester der Formel (Id),

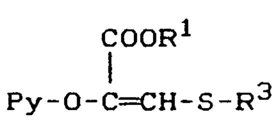 (Id)

in welcher

$R^1$, $R^3$ und Py die oben angegebene Bedeutung haben, erhält,
indem man substituierte Acrylsäureester der Formel (VIII),

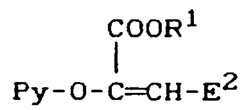

(VIII)

in welcher
$E^2$ für eine elektronenanziehende Abgangsgruppe steht und
$R^1$ und Py die oben angegebene Bedeutung haben,
mit Thiolen der Formel (IX),

$R^3$ - SH (IX)

in welcher
$R^3$ die oben angegebene Bedeutung hat,
gegebenenfalls in Gegenwart eines Verdünnungsmittels und gegebenenfalls in Gegenwart eines Reaktionshilfsmittels umsetzt.

**5.** Schädlingsbekämpfungsmittel, gekennzeichnet durch einen Gehalt an mindestens einem pyridyl-substituiertem Acrylsäureester der Formel (I) nach den Ansprüchen 1 bis 3.

**6.** Verwendung von pyridyl-substituierten Acrylsäureestern der Formel (I) nach den Ansprüchen 1 bis 3 zur Bekämpfung von Schädlingen.

**7.** Verfahren zur Bekämpfung von Schädlingen, dadurch gekennzeichnet, daß man pyridyl-substituierte Acrylsäureester der Formel (I) nach den Ansprüchen 1 bis 3 auf Schädlinge und/oder ihren Lebensraum einwirken läßt.

**8.** Verfahren zur Herstellung von Schädlingsbekämpfungsmitteln, dadurch gekennzeichnet, daß man pyridyl-substituierte Acrylsäureester der Formel (I) nach den Ansprüchen 1 bis 3 mit Streckmitteln und/oder oberflächenaktiven Mitteln vermischt.

**9.** Hydroxyacrylsäureester der allgemeinen Formel (II a)

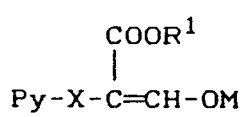

(II a)

in welcher
$R^1$, Py und X die in Anspruch 1 angegebene Beckutung haben und
M für Wasserstoff oder für ein Alkalimetallkation steht,
ausgenommen die Verbindung 2-(6-Methylpyrid-3-yloxy)-3-hydroxyacrylsäureethylester.

**10.** Verfahren zur Herstellung von Hydroxyacrylsäureestern der allgemeinen Formel (II a)

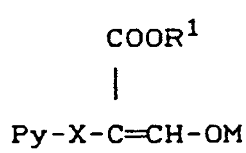

(II a)

in welcher

$R^1$, X, Py und M die in Anspruch 9 angegebene Bedeutung haben,
ausgenommen die Verbindung 2-(6-Methylpyrid-3-yloxy)-3-hydroxyacrylsäureethylester, dadurch gekennzeichnet, daß man substituierte Essigsäureester der Formel (IV),

$Py\text{-}X\text{-}CH_2\text{-}COOR^1$ (IV)

in welcher
$R^1$, Py, und X die oben angegebene Bedeutung haben, ausgenommen die Verbindung 2-(6-Methylpyrid-3-yloxy)-essigsäureethylester,
mit Ameisensäureestern der Formel (X),

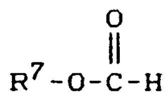
(X)

in welcher
$R^7$ für Alkyl steht,
gegebenenfalls in Gegenwart eines Verdünnungsmittels und gegebenenfalls in Gegenwart eines basischen Reaktionshilfsmittels bei Temperaturen zwischen -20 °C und +50 °C umsetzt.

**11.** Pyridyl-substituierte Acrylsäureester der Formel (VIII)

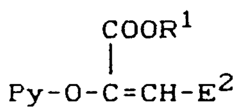
(VIII)

in welcher
$R^1$ und Py die in Anspruch 1 angegebene Bedeutung haben, und
$E^2$ für eine elektronenanziehende Abgangsgruppe steht.

**12.** Verfahren zur Herstellung von pyridyl-substituierten Acrylsäureestern der Formel (VIII)

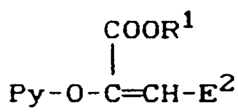
(VIII)

in welcher
$R^1$, Py und E die in Anspruch 11 angegebene Bedeutung haben, dadurch gekennzeichnet, daß man Hydroxyacrylsäureester der Formel (IIb)

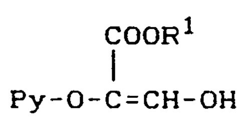
(IIb)

in welcher
$R^1$ und Py die oben angegebene Bedeutung haben,
mit Säurechloriden der Formel (XIII),

$R^8\text{-}Cl$ (XIII)

in welcher

$R^8$ für einen Acyl- oder Sulfonylrest steht,
gegebenenfalls in Gegenwart eines Verdünnungsmittels und gegebenenfalls in Gegenwart eines Säurebindemittels bei Temperaturen zwischen -20 °C und + 120 °C umsetzt.

**13.** Verwendung der Verbindungen der Formeln (IIa) und (VIII) gemäß den Ansprüchen 9 und 11 als Ausgangsverbindungen.

**14.** Essigsäure der Formel (IVa)

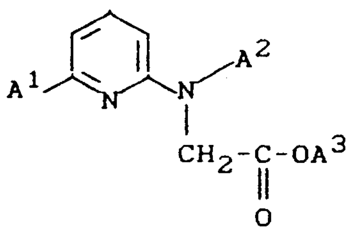

(IVa)

in welcher

$A^1$ für jeweils unsubstituiertes oder einfach bis mehrfach, gleich oder verschieden substituiertes Phenyl, Naphthyl, Pyridyl, Primidinyl, Pyrazinyl, Pyridazinyl, Thienyl, Furanyl, Pyrrolyl, Thiazolyl, Pyrazolyl oder Oxazolyl steht, wobei als Substituenten jeweils genannt seien:
Halogen, Methyl, Ethyl, n- oder i-Propyl, n-, i-, s- oder t-Butyl, Methoxy, Ethoxy, n- oder i-Propoxy, n- i-, s- oder t-Butoxy, Halogenalkyl mit 1 oder 2 Kohlenstoffatomen und 1 bis 5 gleichen oder verschiedenen Fluor-oder Chloratomen, Methylthio, Ethylthio, n-oder i-Propylthio, n-, i-, s-oder t-Butylthio, Alkylidendioxy mit 1 bis 3 Kohlenstoffatomen, unsubstituiertes oder einfach bis mehrfach, gleich oder verschieden durch Methyl, Ethyl, Methoxy, Ethoxy, Halogen oder Halogenmethyl mit 1 bis 3 gleichen oder verschiedenen Fluor oder Chloratomen substituiertes Phenyl,

$A^1$ steht weiterhin für unsubstituiertes oder einfach bis mehrfach gleich oder verschieden substituiertes Phenoxy, wobei als Substituenten Halogen, Phenoxy, Methyl, Ethyl, n- oder i-Propyl infrage kommen
oder für eine Gruppierung $-C \equiv C-A^4$ oder $C-CH=CH-A^4$,

$A^2$ für Methyl, Ethyl, n- oder i-Propyl, n-, i-, s-oder t-Butyl steht,

$A^3$ für Methyl, Ethyl, n- oder i-Propyl, n-, i-, s-oder t-Butyl steht und

$A^4$ für unsubstituiertes oder substituiertes Phenyl steht, wobei als Substituenten die weiter oben bei $A^1$ angegebenen Substituenten infrage kommen.

**15.** Verfahren zur Herstellung von Essigsäureestern der Formel (IVa)

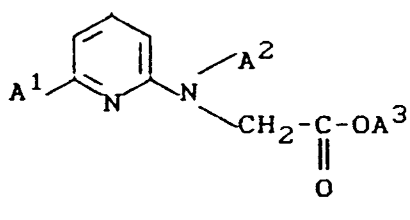

(IVa)

in welcher

$A^1$ für jeweils unsubstituiertes oder einfach bis mehrfach, gleich oder verschieden substituiertes Phenyl, Naphthyl, Pyridyl, Primidinyl, Pyrazinyl, Pyridazinyl, Thienyl, Furanyl, Pyrrolyl, Thiazolyl, Pyrazolyl oder Oxazolyl steht, wobei als Substituenten jeweils genannt seien:
Halogen, Methyl, Ethyl, n- oder i-Propyl, n-, i-, s- oder t-Butyl, Methoxy, Ethoxy, n- oder i-Propoxy, n- i-, s- oder t-Butoxy, Halogenalkyl mit 1 oder 2 Kohlenstoffatomen und 1 bis 5 gleichen oder verschiedenen Fluor-oder Chloratomen, Methylthio, Ethylthio, n-oder i-Propylthio, n-, i-, s-oder t-Butylthio, Alkylidendioxy mit 1 bis 3 Kohlenstoffatomen, unsubstituiertes

oder einfach bis mehrfach, gleich oder verschieden durch Methyl, Ethyl, Methoxy, Ethoxy, Halogen oder Halogenmethyl mit 1 bis 3 gleichen oder verschiedenen Fluor oder Chloratomen substituiertes Phenyl,

$A^1$ steht weiterhin für unsubstituiertes oder einfach bis mehrfach gleich oder verschieden substituiertes Phenoxy, wobei als Substituenten Halogen, Phenoxy, Methyl, Ethyl, n- oder i-Propyl infrage kommen
oder für eine Gruppierung $-C \equiv C-A^4$ oder $C-CH=CH-A^4$,

$A^2$ für Methyl, Ethyl, n- oder i-Propyl, n-, i-, s-oder t-Butyl steht,

$A^3$ für Methyl, Ethyl, n- oder i-Propyl, n-, i-, s-oder t-Butyl steht und

$A^4$ für unsubstituiertes oder substituiertes Phenyl steht, wobei als Substituenten die weiter oben bei $A^1$ angegebenen Substituenten infrage kommen,

dadurch gekennzeichnet, daß man

α) substituierte Halogenpyridine der Formel (XIV)

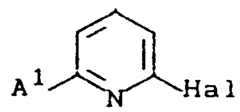

(XIV)

in welcher

$A^1$ die oben angegebene Bedeutung hat und

Hal für Chlor oder Brom steht,

mit Aminosäurederivaten der Formel (XV)

$A^2-NH-CH_2-COOA^3$ (XV)

in welcher

$A^2$ und $A^3$ die oben angegebene Bedeutung haben,

gegebenenfalls in Gegenwart eines Verdünnungsmittels und gegebenenfalls in Gegenwart einer Base umsetzt,

oder wenn man

β) substituierte Aminopyridine der Formel (XVI)

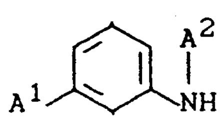

(XVI)

in welcher

$A^1$ und $A^2$ die oben angegebene Bedeutung haben,

mit Halogencarbonsäureestern der Formel (XVII)

$Hal-CH_2-COOA^3$ (XVII)

in welcher

$A^3$ die oben angegebene Bedeutung hat und

Hal für Chlor oder Brom steht,

gegebenenfalls in Gegenwart eines Verdünnungsmittels und gegebenenfalls in Gegenwart einer Base umsetzt.

**16.** Schädlingsbekämpfungsmittel, gekennzeichnet durch einen Gehalt an mindestens einer Verbindung der Formel (IVa) nach Anspruch 14.

**17.** Verwendung von Verbindungen der Formel (IVa) nach Anspruch 14 zur Bekämpfung von Schädlingen.

## Claims

**1.** Pyridyl-substituted acrylic esters of the general formula (I)

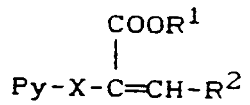

(I)

in which

$R^1$ represents straight-chain or branched alkyl which has 1 to 6 carbon atoms, or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, suitable aryl substituents being: halogen, cyano, nitro, in each case straight-chain or branched alkyl, alkoxy or alkylthio, each having 1 to 4 carbon atoms, in each case straight-chain or branched halogenoalkyl, halogenoalkoxy or halogenoalkylthio, each having 1 to 4 carbon atoms and 1 to 9 identical or different halogen atoms, in each case straight-chain or branched alkoxycarbonyl or alkoximinoalkyl, each having 1 to 8 carbon atoms in the individual alkyl moieties, cycloalkyl which has 3 to 7 carbon atoms, double-linked alkanediyl which has 3 to 5 carbon atoms, aryl, aralkyl, aryloxy or aralkyloxy, each of which has 6 to 10 carbon atoms in the aryl moiety and if appropriate 1 to 4 carbon atoms in the straight-chain or branched alkyl moiety and each of which is optionally monosubstituted to polysubstituted in the aryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms, or heteroarylalkyl or heteroaryl, each of which has 2 to 9 carbon atoms and 1 to 4 identical or different hetero atoms in the heteroaryl moiety and if appropriate 1 to 4 carbon atoms in the straight-chain or branched alkyl moiety and each of which is optionally monosubstituted to polysubstituted in the heteroaryl moiety by identical or different substituents from the series comprising halogen, alkyl, alkoxy, alkylthio, halogenoalkyl, halogenoalkoxy or halogenoalkylthio, each having 1 to 4 carbon atoms and if appropriate 1 to 9 identical or different halogen atoms;

$R^2$ represents dialkylamino which has 1 to 6 carbon atoms in each of the individual alkyl moieties, or represents a radical $-Z-R^3$,

X represents oxygen or sulphur or represents a radical

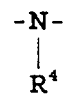

and

Py represents 2-pyridyl, 3-pyridyl or 4-pyridyl, each of which is monosubstituted to polysubstituted by identical or different substituents, suitable substituents in each case being: halogen, cyano, nitro, in each case straight-chain or branched alkyl, alkoxy or alkylthio, each having 1 to 6 carbon atoms, in each case straight-chain or branched halogenoalkyl, halogenoalkoxy or halogenoalkylthio, each having 1 to 4 carbon atoms and 1 to 9 identical or different halogen atoms, in each case straight-chain or branched alkoxycarbonyl, alkoximinoalkyl, dialkylamino or dialkylaminocarbonyl, each having 1 to 4 carbon atoms in the individual alkyl moieties, in each case straight-chain or branched alkenyl or alkinyl, each having 2 to 8 carbon atoms, or aryl, aryloxy, arylthio, arylcarbonyl, aralkyl, aralkenyl, aralkinyl, aralkyloxy or heteroaryl, each of which has 6 to 10 carbon atoms in the aryl moiety and if appropriate 1 to 6 carbon atoms in the straight-chain or branched alkyl moiety or if appropriate 2 to 6 carbon atoms in the straight-chain or branched alkenyl moiety or alkinyl moiety or 2 to 9 carbon atoms and 1 to 4 identical or different hetero atoms in the heteroaryl moiety and each of which is optionally monosubstituted to polysubstituted in the respective

aryl moiety or in the heteroaryl moiety by identical or different substituents from the series comprising halogen, alkyl, alkoxy, alkylthio, halogenoalkyl, halogenoalkoxy, halogenoalkylthio, dioxyalkylene, halogen-substituted dioxyalkylene or optionally substituted phenyl;
where

$R^3$ represents straight-chain or branched alkyl which has 1 to 6 carbon atoms or represents aralkyl which has 1 to 4 carbon atoms in the straight-chain or branched alkyl moiety and 6 to 10 carbon atoms in the aryl moiety and which is optionally monosubstituted to polysubstituted in the aryl moiety by identical or different substituents, suitable substituents being the aryl substituents mentioned in the case of $R^1$,

$R^4$ represents hydrogen, or represents straight-chain or branched alkyl which has 1 to 6 carbon atoms, or represents straight-chain or branched alkanoyl which has 1 to 6 carbon atoms in the alkyl moiety, or represents aralkyl or aryl which has 1 to 6 carbon atoms in the straight-chain or branched alkyl moiety and 6 to 10 carbon atoms in the respective aryl moiety and each of which is optionally monosubstituted to polysubstituted in the respective aryl moiety by identical or different substituents, suitable substituents in the aryl moiety in each case being those mentioned in the case of $R^1$ and

Z represents oxygen or sulphur.

**2.** Pyridyl-substituted acrylic esters of the formula (I) according to Claim 1, where

$R^1$ represents methyl, ethyl, n- or i-propyl or n-, i-, s- or t-butyl, or represents benzyl which is optionally monosubstituted to trisubstituted by identical or different substituents, suitable substituents being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl or 1,4-butanediyl, or phenyl, benzyl, phenoxy or benzyloxy, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio,

$R^2$ represents dialkylamino which has 1 to 4 carbon atoms in each of the individual alkyl moieties, or represents a radical -Z-$R^3$,

X represents oxygen or sulphur or represents a radical

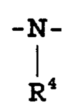

and

Py represents 2-pyridyl, 3-pyridyl or 4-pyridyl, each of which is optionally monosubstituted to trisubstituted by identical or different substituents, at least one substituent in each case representing phenyl, naphthyl, phenoxy, phenylthio, phenylcarbonyl, benzyl, phenylethyl, phenylpropyl, phenylethenyl, benzyloxy or heteroaryl, each of which is optionally monosubstituted to trisubstituted by identical or different substituents from the series comprising fluorine, chlorine, bromine, methyl, ethyl, n- or i-propyl, n-, i-, s- or t-butyl, methoxy, ethoxy, n- or i-propoxy, methylthio, ethylthio, trifluoromethyl, trifluoromethoxy, trifluoromethylthio, dioxymethylene, or by phenyl which is optionally monosubstituted to trisubstituted by identical or different substituents from the series comprising fluorine, chlorine, methyl, methoxy or trifluoromethyl, suitable specific heteroaryl radicals being the following:

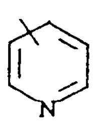 ; 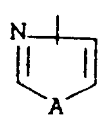 ; 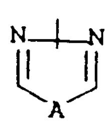 ; 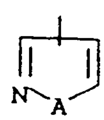 ; 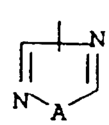 ; 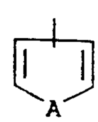 ,

which can also be benzo-fused, if appropriate, and in which

A in each case represents oxygen or sulphur or represents an NH group; other pyridyl substituents are: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trifluoromethylthio, methoxycarbonyl, ethoxycarbonyl, methoximinomethyl, ethoximinomethyl, methoximinoethyl, ethoximinoethyl, dimethylamino, diethylamino, dimethylcarbamoyl, diethylcarbamoyl, allyl, butenyl or propargyl; where

$R^3$ represents methyl, ethyl, n- or i-propyl, or n-, i-, s- or t-butyl, or represents benzyl which is optionally monosubstituted to trisubstituted by identical or different substituents, suitable substituents being those mentioned in the case of $R^1$ and $R^2$;

$R^4$ represents hydrogen, methyl, ethyl, n- or i-propyl or n-, i-, s- or t-butyl, or represents acetyl, propionyl or n- or i-butyryl or represents benzyl or phenyl, each of which is optionally monosubstituted to trisubstituted by identical or different substituents, suitable substituents being those mentioned in the case of $R^1$ and $R^2$, and

Z represents oxygen or sulphur.

**3.** Pyridyl-substituted acrylic acid of the formula (I) according to Claim 1, where

$R^1$ represents methyl or ethyl

$R^2$ represents methoxy or ethoxy,

X represents oxygen or sulphur or represents a radical

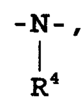

Py represents 2-pyridyl, 3-pyridyl or 4-pyridyl, each of which is optionally monosubstituted to trisubstituted by identical or different substituents, at least one substituent in each case representing benzo-fused phenyl, phenoxy, phenylcarbonyl, benzyl, pyridyl, pyrazolyl, oxazolyl, thiazolyl, thienyl, furyl, thiadiazolyl, oxadiazolyl, imidazolyl or triazolyl, each of which is optionally monosubstituted to trisubstituted by identical or different substituents from the series comprising fluorine, chlorine, methyl, ethyl, methoxy, trifluoromethyl, dioxymethylene or phenyl, suitable further pyridyl substituents in each case being: fluorine, chlorine, bromine, cyano, nitro, methyl, ethyl, methoxy, methylthio, trifluoromethyl, trifluoromethoxy, trifluoromethylthio, methoxycarbonyl, ethoxycarbonyl or dimethylamino, and where

$R^4$ represents methyl, ethyl or benzyl.

**4.** Process for the preparation of pyridyl-substituted acrylic esters of the general formula (I)

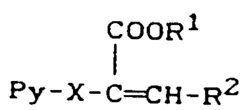

(I)

in which

$R^1$, $R^2$, X and Py have the meaning given in Claim 1,

characterized in that:

(a) pyridyl-substituted acrylic esters of the formula (Ia)

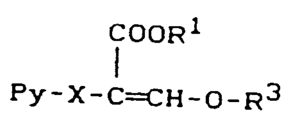

(Ia)

in which

$R^1$, $R^3$, X and Py have the abovementioned meaning

are obtained by reacting hydroxyacrylic esters or their alkali metal salts of the formula (II)

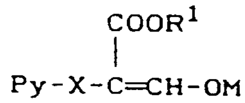

(II)

in which

M represents hydrogen or represents an alkali metal cation and

$R^1$, X and Py have the abovementioned meaning

with alkylating agents of the formula (III)

$R^3$-$E^1$ (III)

in which

$E^1$ represents an electron-withdrawing leaving group and

$R^3$ has the abovementioned meaning,

if appropriate in the presence of a diluent and if appropriate in the presence of a reaction auxiliary, or

(b) pyridyl-substituted acrylic esters of the formula (Ib)

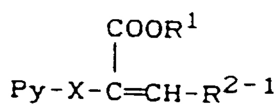

(Ib)

in which

$R^{2-1}$ represents dialkylamino and

$R^1$, X and Py have the abovementioned meaning

are obtained by reacting substituted acetic esters of the formula (IV)

Py-X-$CH_2$-$COOR^1$ (IV)

in which

$R^1$, X and Py have the abovementioned meaning

with formamides of the formula (Va)

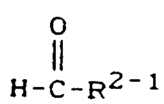

(Va)

in which

$R^{2-1}$ has the abovementioned meaning,

or with derivatives of these formamides, of the formula (Vb)

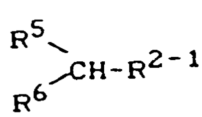

(Vb)

in which

$R^5$ and $R^6$ independently of one another each represent alkoxy or dialkylamino and

$R^{2-1}$ has the abovementioned meaning,

if appropriate in the presence of a diluent; or
c) pyridyl-substituted acrylic esters of the formula (Ic)

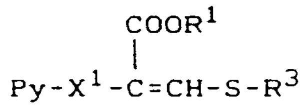

(Ic)

in which
$X^1$ represents sulphur or represents a radical

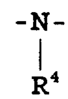

and
$R^1$, $R^3$ and Py have the abovementioned meaning
are obtained by reacting oxalic acid derivatives of the formula (VI)

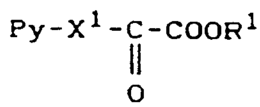

(VI)

in which
$R^1$, $X^1$ and Py have the abovementioned meaning
with organometal compounds of the formula (VII)

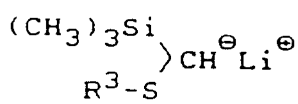

(VII)

in which
$R^3$ has the abovementioned meaning,
if appropriate in the presence of a diluent; or
(d) pyridyl-substituted acrylic esters of the formula (Id)

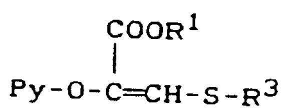

(Id)

in which
$R^1$, $R^3$ and Py have the abovementioned meaning
are obtained by reacting substituted acrylic esters of the formula (VIII)

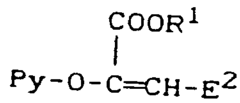

(VIII)

in which

$E^2$ represents an electron-withdrawing leaving group and

$R^1$ and Py have the abovementioned meaning

with thiols of the formula (IX)

$R^3$ - SH (IX)

in which

$R^3$ has the abovementioned meaning,

if appropriate in the presence of a diluent and if appropriate in the presence of a reaction auxiliary.

**5.** Pesticides, characterized in that they contain at least one pyridyl-substituted acrylic ester of the formula (I) according to Claims 1 to 3.

**6.** Use of pyridyl-substituted acrylic esters of the formula (I) according to Claims 1 to 3 for combating pests.

**7.** Method of combating pests, characterized in that pyridyl-substituted acrylic esters of the formula (I) according to Claims 1 to 3 are allowed to act on pests and/or their environment.

**8.** Process for the preparation of pesticides, characterized in that pyridyl-substituted acrylic esters of the formula (I) according to Claims 1 to 3 are mixed with extenders and/or surface-active agents.

**9.** Hydroxyacrylic esters of the general formula (II a)

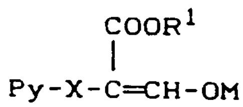

(II a)

in which

$R^1$, Py and X have the meaning given in Claim 1 and

M represents hydrogen or represents an alkali metal cation,

with the exception of the compound ethyl 2-(6-methylpyrid-3-yloxy)-3-hydroxyacrylate.

**10.** Process for the preparation of hydroxyacrylic esters of the general formula (II a)

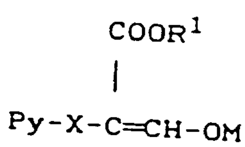

(II a)

in which

$R^1$, X, Py and M have the meaning given in Claim 9,

with the exception of the compound ethyl 2-(6-methylpyrid-3-yloxy)-3-hydroxyacrylate, characterized in that substituted acetic esters of the formula (IV)

Py-X-$CH_2$-$COOR^1$ (IV)

in which

$R^1$, Py and X have the abovementioned meaning, with the exception of the compound ethyl 2-(6-methylpyrid-3-yloxy)-acetate,

are reacted with formic esters of the formula (X)

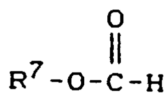

(X)

in which

$R^7$ represents alkyl,

if appropriate in the presence of a diluent and if appropriate in the presence of a basic reaction auxiliary, at temperatures between -20 °C and +50 °C.

**11.** Pyridyl-substituted acrylic esters of the formula (VIII)

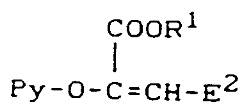

(VIII)

in which

$R^1$ and P y have the meaning given in Claim 1, and

$E^2$ represents an electron-withdrawing leaving group.

**12.** Process for the preparation of pyridyl-substituted acrylic esters of the formula (VIII)

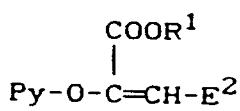

(VIII)

in which

$R^1$, Py and E have the meaning given in Claim 11,

characterized in that hydroxyacrylic esters of the formula (IIb)

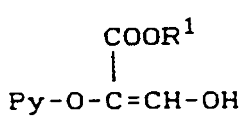

(IIb)

in which

$R^1$ and Py have the abovementioned meaning

are reacted with acid chlorides of the formula (XIII)

$R^8$-Cl (XIII)

in which

$R^8$ represents an acyl radical or sulphonyl radical,

if appropriate in the presence of a diluent and if appropriate in the presence of an acid-binding agent, at temperatures between -20 °C and +120 °C.

**13.** Use of the compounds of the formulae (IIa) and (VIII) according to Claims 9 and 11 as starting compounds.

**14.** Acetic acid of the formula (IVa)

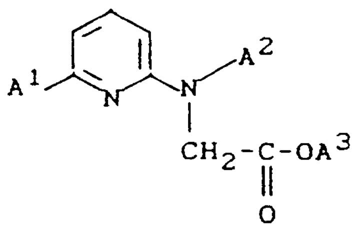

(IVa)

in which

$A^1$ represents phenyl, naphthyl, pyridyl, pyrimidinyl, pyrazinyl, pyridazinyl, thienyl, furanyl, pyrrolyl, thiazolyl, pyrazolyl or oxazolyl, each of which is optionally monosubstituted to polysubstituted by identical or different substituents, suitable substituents in each case being: Halogen, methyl, ethyl, n- or i-propyl, n-, i-, s- or t-butyl, methoxy, ethoxy, n- or i-propoxy, n-, i-, s- or t-butoxy, halogenoalkyl having 1 or 2 carbon atoms and 1 to 5 identical or different fluorine or chlorine atoms, methylthio, ethylthio, n- or i-propylthio, n-, i-, s- or t-butylthio, alkylidenedioxy having 1 to 3 carbon atoms, phenyl which is optionally monosubstituted to polysubstituted by identical or different substituents from the series comprising methyl, ethyl, methoxy, ethoxy, halogen or halogenomethyl having 1 to 3 identical or different fluorine or chlorine atoms,

$A^1$ furthermore represents phenoxy which is optionally monosubstituted to polysubstituted by identical or different substituents, suitable substituents being halogen, phenoxy, methyl, ethyl and n- or i-propyl or represents a group $-C{\equiv}C-A^4$ or $C-CH=CH-A^4$,

$A^2$ represents methyl, ethyl, n- or i-propyl or n-, i-, s- or t-butyl,

$A^3$ represents methyl, ethyl, n- or i-propyl or n-, i-, s- or t-butyl and

$A^4$ represents optionally substituted phenyl, suitable substituents being the substituents given further above in the case of $A^1$.

**15.** Process for the preparation of acetic esters of the formula (IVa)

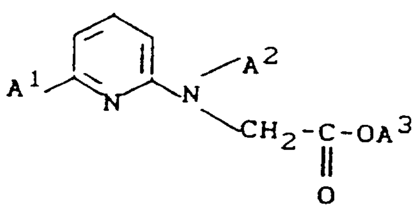

(IVa)

in which

$A^1$ represents phenyl, naphthyl, pyridyl, pyrimidinyl, pyrazinyl, pyridazinyl, thienyl, furanyl, pyrrolyl, thiazolyl, pyrazolyl or oxazolyl, each of which is optionally monosubstituted to polysubstituted by identical or different substituents, suitable substituents in each case being: Halogen, methyl, ethyl, n- or i-propyl, n-, i-, s- or t-butyl, methoxy, ethoxy, n- or i-propoxy, n-, i-, s- or t-butoxy, halogenoalkyl having 1 or 2 carbon atoms and 1 to 5 identical or different fluorine or chlorine atoms, methylthio, ethylthio, n- or i-propylthio, n-, i-, s- or t-butylthio, alkylidenedioxy having 1 to 3 carbon atoms, phenyl which is optionally monosubstituted to polysubstituted by identical or different substituents from the series comprising methyl, ethyl, methoxy, ethoxy, halogen or halogenomethyl having 1 to 3 identical or different fluorine or

chlorine atoms,

$A^1$ furthermore represents phenoxy which is optionally monosubstituted to polysubstituted by identical or different substituents, suitable substituents being halogen, phenoxy, methyl, ethyl and n- or i-propyl or represents a group -C≡C-$A^4$ or C-CH=CH-$A^4$,

$A^2$ represents methyl, ethyl, n- or i-propyl or n-, i-, s- or t-butyl,

$A^3$ represents methyl, ethyl, n- or i-propyl or n-, i-, s- or t-butyl and

$A^4$ represents optionally substituted phenyl, suitable substituents being the substituents given further above in the case of $A^1$,

characterized in that

α) substituted halogenopyridines of the formula (XIV)

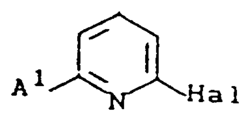

(XIV)

in which

$A^1$ has the abovementioned meaning and

Hal represents chlorine or bromine

are reacted with amino acid derivatives of the formula (XV)

$A^2$-NH-$CH_2$-$COOA^3$ (XV)

in which

$A^2$ and $A^3$ have the abovementioned meaning,

if appropriate in the presence of a diluent and if appropriate in the presence of a base,

or when

β) substituted aminopyridines of the formula (XVI)

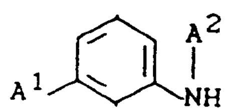

(XVI)

in which

$A^1$ and $A^2$ have the abovementioned meaning are reacted with halogenocarboxylic esters of the formula (XVII)

Hal-$CH_2$-$COOA^3$ (XVII)

in which

$A^3$ has the abovementioned meaning and

Hal represents chlorine or bromine,

if appropriate in the presence of a diluent and if appropriate in the presence of a base.

**16.** Pesticides, characterized in that they contain at least one compound of the formula (IVa) according to Claim 14.

**17.** Use of compounds of the formula (IVa) according to Claim 14 for combating pests.

## Revendications

**1.** Ester d'acide acrylique à substituant pyridyle, de formule générale (I)

```
        COOR1
        |
Py-X-C=CH-R2                    (I)
```

dans laquelle

- $R^1$ est un groupe alkyle linéaire ou ramifié ayant 1 à 6 atomes de carbone ou un groupe aralkyle, éventuellement substitué une ou plusieurs fois identiques ou différentes dans la partie aryle, ayant 1 à 4 atomes de carbone dans la partie alkyle linéaire ou ramifiée et 6 à 10 atomes de carbone dans la partie aryle, en considérant comme substituants de la partie aryle : un halogène, un radical cyano, nitro, un radical alkyle, alkoxy ou alkylthio linéaire ou ramifié ayant dans chaque cas 1 à 4 atomes de carbone, un radical halogénalkyle, halogénalkoxy ou halogénalkylthio linéaire ou ramifié avec à chaque fois 1 à 4 atomes de carbone et 1 à 9 atomes d'halogènes identiques ou différents, un radical alkoxycarbonyle ou alkoximinoalkyle linéaire ou ramifié avec dans chaque cas 1 à 8 atomes de carbone dans les parties alkyle individuelles, un groupe cycloalkyle de 3 à 7 atomes de carbone, un groupe alcanediyle doublement lié ayant 3 à 5 atomes de carbone, un groupe aryle, aralkyle, aryloxy ou aralkyloxy portant chacun le cas échéant dans la partie aryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents, chacun ayant 6 à 10 atomes de carbone dans la partie aryle et le cas échéant 1 à 4 atomes de carbone dans la partie alkyle linéaire ou ramifiée, ou bien un groupe hétéroarylalkyle ou hétéroaryle portant chacun le cas échant dans la partie hétéroaryle un ou plusieurs substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents, chacun ayant 2 à 9 atomes de carbone et 1 à 4 hétéroatomes identiques ou différents dans la partie hétéroaryle et le cas échéant 1 à 4 atomes de carbone dans la partie alkyle linéaire ou ramifiée ;
- $R^2$ est un groupe dialkylamino ayant 1 à 6 atomes de carbone dans chacune des parties alkyle, ou un reste $-Z-R^3$,
- X représente de l'oxygène, du soufre ou un reste

```
-N-
 |
 R4
```

et

- Py représente un groupe 2-pyridyle, 3-pyridyle ou 4-pyridyle portant chacun un ou plusieurs substituants identiques ou différents, en considérant dans chaque cas comme substituants : un halogène, un radical cyano, nitro, un groupe alkyle, alkoxy ou alkylthio linéaire ou ramifié ayant chacun 1 à 6 atomes de carbone, un groupe halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle, alkoximinoalkyle, dialkylamino ou dialkylaminocarbonyle linéaire ou ramifié ayant chacun 1 à 4 atomes de carbone dans les parties alkyle individuelles, un groupe alcényle ou alcynyle linéaire ou ramifié ayant chacun 2 à 8 atomes de carbone ou un groupe aryle, aryloxy, arylthio, arylcarbonyle, aralkyle, aralcényle, aralcynyle, aralkyloxy ou hétéroaryle portant chacun le cas, dans la partie aryle ou dans la partie hétéroaryle, un ou plusieurs substituants, identiques ou différents, halogéno, alkyle, alkoxy, alkylthio, halogénalkyle, halogénalkoxy, halogénalkylthio, dioxyalkylène, dioxyalkylène à substituant halogéno ou phényle éventuellement substitué, ayant chacun 6 à 10 atomes de carbone dans la partie aryle et le cas échéant 1 à 6 atomes

de carbone dans la partie alkyle linéaire ou ramifiée ou le cas échéant 2 à 6 atomes de carbone dans la partie alcényle ou alcynyle linéaire ou ramifiée, ou avec 2 à 9 atomes de carbone et 1 à 4 hétéroatomes identiques ou différents dans la partie hétéroaryle ;

$R^3$ représentant un groupe alkyle linéaire ou ramifié ayant 1 à 6 atomes de carbone ou un groupe aralkyle, éventuellement substitué dans la partie aryle une ou plusieurs fois identiques ou différentes, ayant 1 à 4 atomes de carbone dans la partie alkyle linéaire ou ramifiée et 6 à 10 atomes de carbone dans la partie aryle, en considérant comme substituants de la partie aryle ceux qui ont été mentionnés pour $R^1$,

$R^4$ représentant de l'hydrogène, un groupe alkyle linéaire ou ramifié ayant 1 à 6 atomes de carbone, un groupe alcanoyle linéaire ou ramifié ayant 1 à 6 atomes de carbone dans la partie alkyle, ou un groupe aralkyle ou aryle éventuellement substitué chacun dans la partie aryle une ou plusieurs fois identiques ou différentes, ayant 1 à 6 atomes de carbone dans la partie alkyle linéaire ou ramifiée et 6 à 10 atomes de carbone dans chaque partie aryle, en considérant comme substituants de la partie aryle ceux qui ont été mentionnés pour $R^1$ et

Z représente de l'oxygène ou du soufre.

**2.** Esters d'acide acrylique à substituant pyridyle de formule (I) suivant la revendication 1, dans lesquels

$R^1$ est un groupe méthyle, éthyle, n-propyle, iso-propyle, n-butyle, isobutyle, sec.-butyle ou tertio-butyle ou un groupe benzyle portant éventuellement un à trois substituants identiques ou différents, en considérant comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butanediyle ou un groupe phényle, benzyle, phénoxy ou benzyloxy, chacun portant éventuellement dans la partie phényle 1 à 3 substituants, identiques ou différents, fluoro, chloro, bromo, méthyle, éthyle, méthoxy, éthoxy, méthylthio, trifluorométhyle, difluorométhoxy, trifluorométhoxy et/ou trifluorométhylthio,

$R^2$ est un groupe dialkylamino ayant 1 à 4 atomes de carbone dans chacune des parties alkyle, ou un reste $-Z-R^3$,

X représente de l'oxygène, du soufre ou un reste

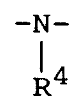

et

Py est un groupe 2-pyridyle, 3-pyridyle ou 4-pyridyle portant chacun le cas échéant un à trois substituants identiques ou différents, au moins un substituant étant un groupe phényle, naphtyle, phénoxy, phénylthio, phénylcarbonyle, benzyle, phényléthyle, phénylpropyle, phényléthényle, benzyloxy ou hétéroaryle portant le cas échéant un à trois substituants, identiques ou différents, fluoro, chloro, bromo, méthyle, éthyle, n-propyle, isopropyle, n-butyle, isobutyle, sec.-butyle, tertio-butyle, méthoxy, éthoxy, n-propoxy, isopropoxy, méthylthio, éthylthio, trifluorométhyle, trifluorométhoxy, trifluorométhylthio, dioxyméthylène ou phényle portant le cas échéant un à trois substituants fluoro, chloro, méthyle, méthoxy ou trifluorométhyle identiques ou différents, en considérant comme restes hétéroaryle en particulier les restes suivants :

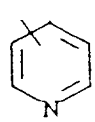 ; 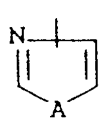 ; 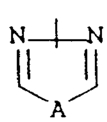 ; 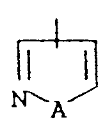 ; 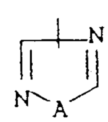 ; 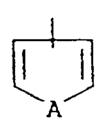 ,

qui peuvent aussi être, le cas échéant, condensés à du benzène et dans lesquels

A représente dans chaque cas un atome d'oxygène, de soufre ou un groupe NH,

d'autres substituants du groupe pyridyle étant : du fluor, du chlore, du brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trifluorométhylthio, méthoxycarbonyle, éthoxycarbonyle, méthoximinométhyle, éthoximinométhyle, méthoximinoéthyle, éthoximinoéthyle, diméthylamino, diéthylamino, diméthylcarbamoyle, diéthylcarbamoyle, allyle, buténylе ou propargyle ;
où

$R^3$ est un groupe méthyle, éthyle, n-propyle, isopropyle, n-butyle, isobutyle, sec.-butyle ou tertio-butyle ou un groupe benzyle portant le cas échéant un à trois substituants identiques ou différents, en considérant comme substituants ceux qui on été mentionnés pour $R^1$ et $R^2$ ;

$R^4$ représente de l'hydrogène, un groupe méthyle, éthyle, n-propyle, isopropyle, n-butyle, isobutyle, sec.-butyle, tertio-butyle, un groupe acétyle, propionyle, n-butyryle ou isobutyryle ou un groupe benzyle ou phényle portant chacun le cas échéant un à trois substituants identiques ou différents, en considérant comme substituants ceux qui ont été mentionnés pour $R^1$ et $R^2$ et

Z représente de l'oxygène ou du soufre.

**3.** Ester d'acide acrylique à substituant pyridyle de formule (I) suivant la revendication 1, dans lequel

$R^1$ est un groupe méthyle ou éthyle,

$R^2$ est un groupe méthoxy ou éthoxy,

X représente de l'oxygène, du soufre ou un reste

$$\begin{array}{c}-N-,\\ |\\ R^4\end{array}$$

Py est un groupe 2-pyridyle, 3-pyridyle ou 4-pyridyle, chacun portant le cas échéant un à trois substituants identiques ou différents, au moins un substituant étant un groupe phényle, phénoxy, phénylcarbonyle, benzyle, pyridyle, pyrazolyle, oxazolyle, thiazolyle, thiényle, furyle, thiadiazolyle, oxadiazolyle, imidazolyle ou triazolyle portant le cas échéant un à trois substituants, identiques ou différents, fluoro, chloro, méthyle, éthyle, méthoxy, trifluorométhyle, dioxyméthylène ou phényle et/ou étant condensés à du benzène, et on considère alors comme autres substituants du groupe pyridyle, dans chaque cas : du fluor, du chlore, du brome, un radical cyano, nitro, méthyle, éthyle, méthoxy, méthylthio, trifluorométhyle, trifluorométhoxy, trifluorométhylthio, méthoxycarbonyle, éthoxycarbonyle ou diméthylamino et

$R^4$ représentant un groupe méthyle, éthyle ou benzyle.

**4.** Procédé de production d'esters d'acide acrylique à substituant pyridyle, de formule générale (I)

$$\begin{array}{c}\quad COOR^1\\ |\\ Py-X-C=CH-R^2\end{array} \qquad (I)$$

dans laquelle

$R^1$, $R^2$, X et Py ont la définition indiquée dans la revendication 1,

caractérisé en ce que :

(a) on obtient des esters d'acide acrylique à substituant pyridyle de formule (Ia)

$$\begin{array}{c}\quad COOR^1\\ |\\ Py-X-C=OH-O-R^3\end{array} \qquad (Ia)$$

dans laquelle

$R^1$, $R^3$, X et Py  ont la définition indiquée ci-dessus

en faisant réagir des esters d'acide hydroxyacrylique ou leurs sels de métaux alcalins de formule (II),

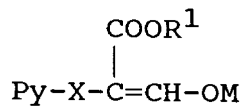

(II)

dans laquelle

M  est de l'hydrogène ou un cation de métal alcalin et

$R^1$, X et Py  ont la définition indiquée ci-dessus,

avec des agents alkylants de formule (III),

$R^3$-$E^1$  (III)

dans laquelle

$E^1$  est un groupe partant attirant les électrons et

$R^3$  a la définition indiquée ci-dessus,

le cas échéant en présence d'un diluant et en la présence éventuelle d'une substance auxiliaire de réaction, ou bien

(b) on obtient des esters d'acide acrylique à substituant pyridyle de formule (Ib),

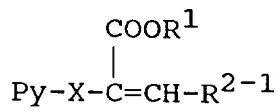

(Ib)

dans laquelle

$R^{2-1}$  est un groupe dialkylamino et

$R^1$, X et Py  ont la définition indiquée ci-dessus,

en faisant réagir des esters d'acide acétique substitués de formule (IV),

Py-X-$CH_2$-$COOR^1$  (IV)

dans laquelle

$R^1$, X et Py  ont la définition indiquée ci-dessus,

avec des formamides de formule (Va),

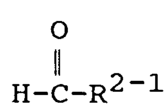

(Va)

dans laquelle

$R^{2-1}$  a la définition indiquée ci-dessus,

ou avec des dérivés de ces formamides de formule (Vb),

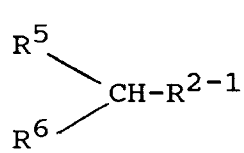

(Vb)

dans laquelle

$R^5$ et $R^6$ représentent dans chaque cas, indépendamment l'un de l'autre, un groupe alkoxy ou dialkylamino et

$R^{2-1}$ a la définition indiquée ci-dessus,

éventuellement en présence d'un diluant, ou bien

(c) on obtient les esters d'acide acrylique à substituant pyridyle de formule (Ic),

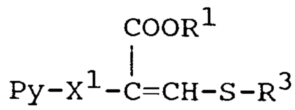

(Ic)

dans laquelle

$X^1$ représente du soufre ou un reste

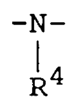

et

$R^1$, $R^3$ et Py ont la définition indiquée ci-dessus,

en faisant réagir des dérivés d'acide oxalique de formule (VI),

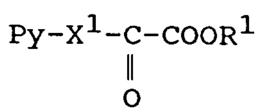

(VI)

dans laquelle

$R^1$, $X^1$ et Py ont la définition indiquée ci-dessus,

avec des composés organométalliques de formule (VII),

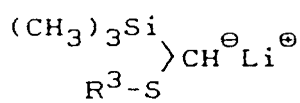

(VII)

dans laquelle

$R^3$ a la définition indiquée ci-dessus,

le cas échéant en présence d'un diluant, ou bien

(d) on obtient des esters d'acide acrylique à substituant pyridyle de formule (Id),

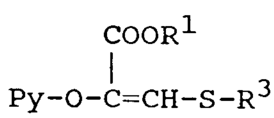

(Id)

dans laquelle

$R^1$, $R^3$ et Py ont la définition indiquée ci-dessus,

en faisant réagir des esters d'acide acrylique substitués de formule (VIII),

```
        COOR1
        |
Py-O-C=CH-E2                    (VIII)
```

dans laquelle

$E^2$ est un groupe partant attirant les électrons et

$R^1$ et Py ont la définition indiquée ci-dessus,

avec des thiols de formule (IX),

$R^3$ - SH (IX)

dans laquelle

$R^3$ a la définition indiquée ci-dessus,

le cas échéant en présence d'un diluant et en la présence éventuelle d'une substance auxiliaire de réaction.

**5.** Compositionspesticides, caractérisées par une teneur en au moins un ester d'acide acrylique à substituant pyridyle de formule (I) suivant les revendications 1 à 3.

**6.** Utilisation d'esters d'acide acrylique à substituant pyridyle de formule (I) suivant les revendications 1 à 3 pour combattre des parasites.

**7.** Procédé pour combattre des parasites, caractérisé en ce qu'on fait agir des esters d'acide acrylique à substituant pyridyle de formule (I) suivant les revendications 1 à 3 sur des parasites et/ou sur leur habitat.

**8.** Procédé de préparation de compositions pesticides, caractérisé en ce qu'on mélange des esters d'acide acrylique à substituant pyridyle de formule (I) suivant les revendications 1 à 3 avec des diluants et/ou des agents tensio-actifs.

**9.** Esters d'acide hydroxyacrylique de formule générale (IIa)

```
        COOR1
        |
Py-X-C=CH-OM                    (IIa)
```

dans laquelle

$R^1$, Py et X ont la définition indiquée dans la revendication 1 et

M représente de l'hydrogène ou un cation de métal alcalino-terreux,

excepté l'ester éthylique d'acide 2-(6-méthylpyrid-3-yloxy)-3-hydroxyacrylique.

**10.** Procédé de production d'esters d'acide hydroxyacrylique de formule générale (IIa)

```
        COOR1
        |
Py-X-C=CH-OM                    (IIa)
```

dans laquelle

$R^1$, X, Py et M ont la définition indiquée dans la revendication 9,

excepté l'ester éthylique d'acide 2-(6-méthylpyrid-3-yloxy)-3-hydroxyacrylique, caractérisé en ce qu'on fait réagir des esters d'acide acétique substitués de formule (IV),

Py-X-$CH_2$-$COOR^1$ (IV)

dans laquelle

$R^1$, Py et X ont la définition indiquée ci-dessus, excepté l'ester éthylique d'acide 2-(6-méthylpyrid-3-yloxy)acétique,

avec des esters d'acide formique de formule (X),

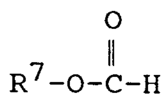

(X)

dans laquelle

$R^7$ est un groupe alkyle,

éventuellement en présence d'un diluant et en la présence éventuelle d'un agent auxiliaire de réaction basique, à des températures comprises entre -20 °C et +50 °C.

**11.** Esters d'acide acrylique à substituant pyridyle, de formule (VIII)

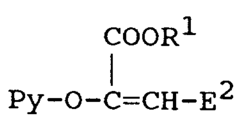

(VIII)

dans laquelle

$R^1$ et Py ont la définition indiquée dans la revendication 1, et

$E^2$ est un groupe partant attirant les électrons.

**12.** Procédé de production d'esters d'acide acrylique à substituant pyridyle, de formule (VIII)

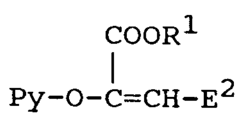

(VIII)

dans laquelle

$R^1$, Py et $E^2$ ont la définition donnée dans la revendication 11,

caractérisé en ce qu'on fait réagir un ester d'acide hydroxyacrylique de formule (IIb)

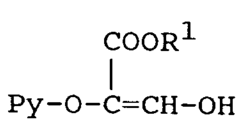

(IIb)

dans laquelle

$R^1$ et Py ont la définition indiquée ci-dessus,

avec des chlorures d'acides de formule (XIII), $R^8$-Cl (XIII) dans laquelle

$R^8$ est un reste acyle ou sulfonyle,

le cas échéant en présence d'un diluant et en la présence éventuelle d'un accepteur d'acide, à des températures comprises entre -20 °C et +120 °C.

**13.** Utilisation des composés de formuls (IIa) et (VIII) suivant les revendications 9 et 11 comme composés de départ.

**14.** Acide acétique de formule (IVa)

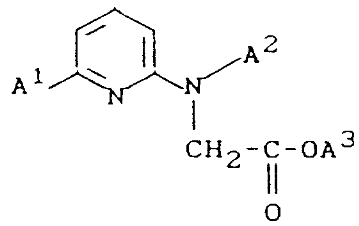

( IVa )

dans laquelle

$A^1$ est un groupe phényle, naphtyle, pyridyle, pyrimidinyle, pyrazinyle, pyridazinyle, thiényle, furannyle, pyrrolyle, thiazolyle, pyrazolyle ou oxazolyle, chacun étant non substitué ou portant un ou plusieurs substituants identiques ou différents, en considérant dans chaque cas comme substituants :
un halogène, un radical méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, halogénalkyle ayant 1 ou 2 atomes de carbone et 1 à 5 atomes de fluor ou de chlore identiques ou différents, méthylthio, éthylthio, n-propylthio, isopropylthio, n-butylthio, isobutylthio, sec.-butylthio, tertiobutylthio, alkylidènedioxy ayant 1 à 3 atomes de carbone, un groupe phényle non substitué ou portant un ou plusieurs substituants, identiques ou différents, méthyle, éthyle, méthoxy, éthoxy, halogéno ou halogénométhyle ayant 1 à 3 atomes de fluor ou de chlore identiques ou différents,

$A^1$ représente en outre un groupe phénoxy non substitué ou portant un ou plusieurs substituants identiques ou différents, en considérant comme substituants un halogène, un radical phénoxy, méthyle, éthyle, n-propyle ou isopropyle, ou bien un groupement $-C{\equiv}C-A^4$ ou $C-CH=CH-A^4$,

$A^2$ est un groupe méthyle, éthyle, n-propyle, isopropyle, n-butyle, isobutyle, sec.-butyle ou tertio-butyle,

$A^3$ est un groupe méthyle, éthyle, n-propyle, isopropyle, n-butyle, isobutyle, sec.-butyle ou tertio-butyle et

$A^4$ est un groupe phényle non substitué ou substitué, en considérant comme substituants les autres substituants indiqués ci-dessus pour $A^1$.

**15.** Procédé de production d'esters d'acide acétique de formule (IVa)

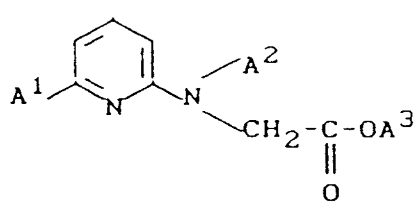

(IVa)

dans laquelle

$A^1$ est un groupe phényle, naphtyle, pyridyle, pyrimidinyle, pyrazinyle, pyridazinyle, thiényle, furannyle, pyrrolyle, thiazolyle, pyrazolyle ou oxazolyle, chacun étant non substitué ou portant un ou plusieurs substituants identiques ou différents, en considérant dans chaque cas comme substituants :
un halogène, un radical méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, halogénalkyle ayant 1 ou 2 atomes de carbone et 1 à 5 atomes de fluor ou de chlore identiques ou différents, méthylthio, éthylthio, n-propylthio, isopropylthio, n-butylthio, isobutylthio, sec.-butylthio, tertiobutylthio, alkylidènedioxy ayant 1 à 3 atomes de carbone, un groupe phényle non substitué ou portant un ou plusieurs substituants, identiques ou différents, méthyle, éthyle, méthoxy, éthoxy, halogéno ou halogénométhyle ayant 1 à 3

atomes de fluor ou de chlore identiques ou différents,

$A^1$ représente en outre un groupe phénoxy non substitué ou portant un ou plusieurs substituants identiques ou différents, en considérant comme substituants un halogène, un radical phénoxy, méthyle, éthyle, n-propyle ou isopropyle, ou bien un groupement $-C\equiv C-A^4$ ou $C-CH=CH-A^4$,

$A^2$ est un groupe méthyle, éthyle, n-propyle, isopropyle, n-butyle, isobutyle, sec.-butyle ou tertio-butyle,

$A^3$ est un groupe méthyle, éthyle, n-propyle, isopropyle, n-butyle, isobutyle, sec.-butyle, tertio-butyle et

$A^4$ est un groupe phényle non substitué ou substitué, en considérant comme substituants les autres substituants indiqués ci-dessus pour $A^1$,

caractérisé en ce que :

α) on fait réagir des halogénopyridines substituées de formule (XIV)

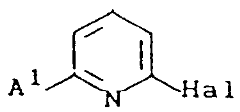

(XIV)

dans laquelle

$A^1$ a la définition indiquée ci-dessus et

Hal représente du chlore ou du brome

avec des dérivés d'aminoacides de formule (XV)

$A^2-NH-CH_2-COOA^3$ (XV)

dans laquelle

$A^2$ et $A^3$ ont la définition indiquée ci-dessus,

éventuellement en présence d'un diluant et en la présence éventuelle d'une base, ou bien

β) on fait réagir des aminopyridines substituées de formule (XVI)

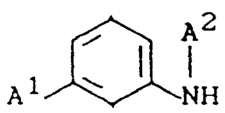

(XVI)

dans laquelle

$A^1$ et $A^2$ ont la définition indiquée ci-dessus,

avec des esters d'acides halogénocarboxyliques de formule (XVII)

$Hal-CH_2-COOA^3$ (XVII)

dans laquelle

$A^3$ a la définition indiquée ci-dessus et

Hal représente du chlore ou du brome,

le cas échéant en présence d'un diluant et en la présence éventuelle d'une base.

**16.** Compositions pesticides, caractérisées par une teneur en au moins un composé de formule (IVa) suivant la revendication 14.

**17.** Utilisation de composés de formule (IVa) suivant la revendication 14 pour combattre des parasites.